# EUROPEAN PATENT SPECIFICATION

(11) **EP 1 858 912 B1**
(45) Date of publication and mention of the grant of the patent: **11.04.2012**
(21) Application number: 06727564.4
(22) Date of filing: 28.02.2006
(51) Int. Cl.: C07K 5/02, A61P 25/28, C07C 233/36, C07F 9/09, C07C 233/47

(54) **COMPOUNDS FOR REDUCING AGGREGATION OF AMYLOID BETA-PEPTIDE**
VERBINDUNGEN ZUR VERMINDERUNG DER AGGREGATION VON AMYLOID-BETA-PEPTID
COMPOSES DESTINES A REDUIRE L'AGREGATION DE BETA-PEPTIDE AMYLOIDE

(30) Priority: 28.02.2005 US 657339 P
(43) Date of publication of application: 28.11.2007
(73) Proprietor: Alphabeta AB, S-182 60 Djursholm (SE)
(72) Inventor: JOHANSSON, Jan, S-116 31 Stockholm (SE); STROMBERG, Roger, S-129 43 Hägersten (SE); NERELIUS, Charlotte, S-75310 Uppsala (SE); LEIJONMARK, Hans, S-113 62 Stockholm (SE); SARGSYAN, Hasmik, Yerevan, Armenia 375010 (AM)
(74) Representative: Bassil, Nicholas Charles
(86) International application number: PCT/IB2006/001112
(87) International publication number: WO 2006/090289

(56) References cited:
- WO-A-01/34631
- WO-A-02/41002
- WO-A-89/08098
- WO-A-98/30229
- WO-A-2006/003877
- DE-A1- 2 136 673
- DE-A1- 2 509 743
- FR-A- 1 381 790
- FR-A- 2 211 557
- US-A- 2 463 779
- US-A- 2 925 639
- US-A1- 2004 147 599
- DATABASE CA [Online] CHEMICAL ABSTRACTS SERVICE, COLUMBUS, OHIO, US; 1989, GAUR, R. K. ET AL: "Fatty acid derivatives of acidic amino acids as potential antibiotics" XP002398870 retrieved from STN Database accession no. 1989:58024 & INDIAN JOURNAL OF CHEMISTRY, SECTION B: ORGANIC CHEMISTRY INCLUDING MEDICINAL CHEMISTRY , 27B(5), 405-8 CODEN: IJSBDB; ISSN: 0376-4699, 1988,
- DATABASE CA [Online] CHEMICAL ABSTRACTS SERVICE, COLUMBUS, OHIO, US; 1975, SUZUKI, TOSHISHIGE ET AL: "Interaction between N-decanoyl diethylentriamine and copper(II) ion in dilute aqueous solutions" XP002398869 retrieved from STN Database accession no. 1975:159993 & KOGYO YOSUI , 195, 18-21 CODEN: KOYOAW; ISSN: 0454-1545, 1974,
- DUPIN, P.; VILORIA-VERA, D. A.; DE SAVIGNAC, A.; LATTES, A.; SUTTER, B.; HAICOUR, P.: "Correlations between the molecular structure of some organic compounds and their corrosion inhibiting properties in deaerated media containing hydrogen sulfide" ANNALI DELL'UNIVERSITA DI FERRARA, SEZIONE 5 - CHIMICA PURA ED APPLICATA, SUPPLEMENTO, vol. 7, 1980, pages 301-322, XP008068964
- ROTHER ENNO; BRANDL RICHARD; BAKER DANIEL L; GOYAL PANKAJ; GEBHARD HARRY; TIGYI GABOR; SIESS WOLFGAN: "Subtype-selective antagonists of lysophosphatidic Acid receptors inhibit platelet activation triggered by the lipid core of atherosclerotic plaques" CIRCULATION, vol. 108, no. 6, 12 August 2003 (2003-08-12), pages 741-747, XP002398863
- GUEGUEN G; GAIGÉ B; GRÉVY J M; ROGALLE P; BELLAN J; WILSON M; KLAÉBÉ A; PONT F; SIMON M F; CHAP H: "Structure-activity analysis of the effects of lysophosphatidic acid on platelet aggregation" BIOCHEMISTRY, vol. 38, no. 26, 29 June 1999 (1999-06-29), pages 8440-8450, XP002398864
- JAN CHUNG-REN; LU YIH-CHAU; JIANN BANG-PING; CHANG HONG-TAI; WANG JUE-LONG; CHEN WEI-CHUNG; HUANG JONG-KHING: "Novel effect of N-palmitoyl-L-serine phosphoric acid on cytosolic Ca2+ levels in human osteoblasts" PHARMACOLOGY & TOXICOLOGY, vol. 93, no. 2, August 2003 (2003-08), pages 71-76, XP002398865
- BITTMAN R; SWORDS B; LILIOM K; TIGYI G: "Inhibitors of lipid phosphatidate receptors: N-palmitoyl-serine and N-palmitoyl-tyrosine phosphoric acids" JOURNAL OF LIPID RESEARCH, vol. 37, no. 2, February 1996 (1996-02), pages 391-398, XP002398866
- TAKEHARA M ET AL: "SURFACE ACTIVE N-ACYLGLUTAMATE: 1. PREPARATION OF LONG CHAIN N-ACYLGLUTAMIC ACID" JOURNAL OF THE AMERICAN OIL CHEMISTS' SOCIETY, AOCS PRESS, CHAMPAIGN, IL, US, vol. 49, 1 March 1972 (1972-03-01), pages 157-161, XP000670144 ISSN: 0003-021X
- VAMVAKIDES A: "EFFET DE QUELQUES DERIVES DU GABA, DE LA GLYCINE OU DE L'ACIDE GLUTAMIQUE SUR LE TEST DE LA NAGE FORCEE CHEZ LA SOURIS" ANNALES PHARMACEUTIQUES FRANCAISES, MASSON, PARIS, FR, vol. 48, no. 3, 1990, pages 154-159, XP002064203 ISSN: 0003-4509
- THOMAS E W; CUDAHY M M; SPILMAN C H; DINH D M; WATKINS T L; VIDMAR T J: "Cholesterol lowering bile acid binding agents: Novel lipophilic polyamines" JOURNAL OF MEDICINAL CHEMISTRY, vol. 35, no. 7, 1992, pages 1233-1245, XP002398946
- VAMVAKIDES ALEXANDRE: "SYNTHESE ET ETUDE PHARMACOLOGIQUE DU PALMITAMIDE ET DU LINOLEAMIDE DE L'ACIDE GLUTAMIQUE" ANNALES PHARMACEUTIQUES FRANCAISES, MASSON, PARIS, FR, vol. 45, no. 5, 1987, pages 389-400, XP008068976 ISSN: 0003-4509

## Description

### TECHNICAL FIELD

This invention relates to compounds and methods for treating amyloidoses.

### BACKGROUND

Alzheimer's disease (AD) is an example of a condition in which specific proteins transform from their native states into bundles of ordered fibrils termed amyloid. Amyloid fibrils are composed of polypeptide chains in β-strand conformation, which form β-sheets running perpendicular to the long axis of the fibril (Serpell, 2000, Biochim. Biophys. Acta 1502:16 30; Makin et al., 2005, Proc. Natl. Acad. Sci. USA 102:315-320. Epub 2005 Jan 03). In general, a 40 or 42 residue Aβ peptide is present in the amyloid plaques that are associated with AD, and formation of Aβ fibrils is thought to be part of the cause of this devastating disease (Selkoe, 2000, J. Am. Med. Assoc. 283, 1615-1617). The post-mortem amounts of Aβ in the cerebral cortices of AD patients correlate with the disease progression, further indicating that Aβ is a rational therapeutic target (Näslund et al., 2000, J. Am. Med. Assoc. 283, 1571-1577). Aβ is generated by proteolytic cleavages of a large transmembrane protein, the amyloid precursor protein (APP). The transmembrane helix of APP is predicted to start at a position corresponding to residue 29 in Aβ. Cleavage by β-secretase is believed to generate the N-terminus of the Aβ peptide, leaving behind a C-terminal membrane-associated stub, which is believed to be cleaved by γ-secretase, generating free Aβ. Cleavage by α-secretase of the C-terminal of residue 16 in Aβ generates a non-amyloidogenic peptide (Esler and Wolfe, 2001, Science 293:1449-1454; Selkoe, 1999, Nature 399:A23-A31). Hereditary forms of early onset AD are associated with point mutations in three regions of APP. Two of these regions are located in close vicinity of the N- and C-terminal ends of Aβ, and are likely associated with elevated production of Aβ(1-40) and/or the more amyloidogenic variant Aβ(1-42). The third region covers positions Ala21, Glu22, and Asp23 of Aβ and the pathogenic mechanisms associated with mutations of these residues are not entirely understood (Haass and Steiner, 2001, Nat. Neurosci. 4:859-860).

### SUMMARY

Compounds (ligands) have been designed that are structurally complementary to one face of the Aβ discordant α-helix and can bind to and stabilize the discordant helix of Aβ. The ability of certain of these ligands to reduce Aβ amyloid fibril formation was confirmed by testing. Such ligands can be useful for treating of Alzheimer's disease (AD) or a related disorder. Also included are compounds that can inhibit the loss of α-helical structures, e.g., of Aβ peptides that are associated with Alzheimer's disease and proteins that include such peptides. Accordingly, the invention relates to a compound that can interact with at least two of the side chain of Lys16 of an Aβ peptide, the side chain of His13 of an Aβ peptide, the side-chain of Glu23 of an Aβ peptide, the side chain of Phe19 of an Aβ peptide, the side chain of Phe20 of an Aβ peptide, and the side-chain of Asp22 of an Aβ peptide; and pharmaceutically acceptable salts thereof. In some aspects, the compound can interact with at least three of the groups (e.g., at least four of the groups). In some aspects, the compound can decrease the loss of α-helix in an Aβ peptide.

The invention provides compounds of Formula 1, 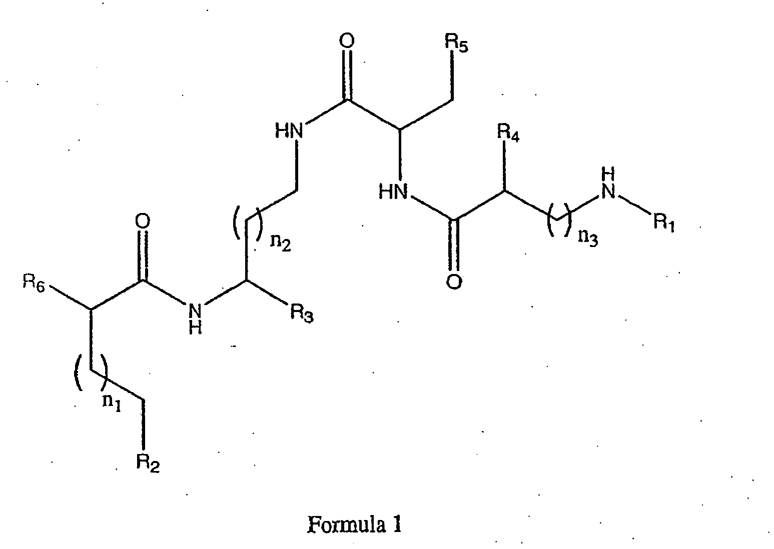 and pharmaceutically acceptable salts and hydrates thereof, wherein:
n₁ is (CH₂)₁₋₄;
n₂ is (CH₂)₁₋₄;
n₃ is (CH₂)₁₋₄;
R₁ is an amine, diamine, -C=NH(NH₂), or carboxylamide
R₂ is carboxylic acid, phosphate, phosphonate, phosphinate, sulfate, or sulfonate; R₃ is carboxylic acid, phosphate, phosphonate, phosphinate, sulfate, or sulfonate; R₄ is an amine, diamine, carboxylic acid, or carboxylamide;
R₃ is an aromatic moiety; and
R₆ is not present or is a carboxylamide or any other group that does not interfere with the association of the compound with the Aβ peptide.

In an embodiment, the compound is ligand 3

In some cases, the invention relates to a composition that includes at least one of the compounds provided herein and a pharmaceutically acceptable excipient, diluent, or carrier.

The invention also relates to a method of decreasing the loss of an Aβ peptide in an α-helical form with the proviso that the method is not a method for treatment of the human or animal body by surgery, or therapy and diagnostic methods practiced on the human or animal body. The method includes contacting an Aβ peptide with a ligand of Formula 1, as provided herein. In another embodiment, the invention relates to a method of decreasing the amount of Aβ in a β form with the proviso that the method is not a method for treatment of the human or animal body by surgery, or therapy and diagnostic methods practiced on the human or animal body. The method includes contacting an Aβ peptide with a ligand as provided herein.

In yet another embodiment, the invention relates to a method of decreasing the formation of Aβ aggregates with the proviso that the method is not a method for treatment of the human or animal body by surgery, or therapy and diagnostic methods practiced on the human or animal body. The method includes contacting an Aβ peptide with a ligand as provided herein.

The invention relates to a compound of formula 1 as provided herein for decreasing the loss of an Aβ peptide in an α-helical form, for decreasing the amount of Aβ in a β form and for decreasing the formation of Aβ aggregates

In one embodiment, the invention can be a method of identifying a compound that is a candidate compound for treating an Aβ peptide-related disorder. The method includes providing a compound (ligand) as provided herein, contacting an Aβ peptide with the compound (thereby providing a sample), determining the amount of Aβ peptide in α-helical form or β form in the sample, comparing the amount of Aβ peptide in α-helical form or β form compared to a reference, such that a compound that increases the amount of Aβ peptide in α-helical form or decreases the amount of Aβ peptide in β form in the sample compared to the reference is a candidate compound. The amount of Aβ peptide in β form is determined, in some cases, using Thioflavin T. In some aspects, the Aβ peptide is provided *in vitro.* In some cases, the Aβ peptide is provided in an animal, e.g., a mouse, rat, *Drosophila,* or a human.

In another embodiment, the invention relates to a therapeutically effective amount of a compound for formula (I) for treating a subject at risk for or having an Aβ peptide-related disorder. The subject can be at risk for or having an Aβ peptide-related disorder (e.g. Alzheimer's disease, Down syndrome, dementia pugilistica, or severe head trauma). In some aspects of the invention, the subject is a mammal (e.g. a human).

An "Aβ peptide" is generally an Aβ peptide that includes the discordant helix of an amyloid precursor protein (APP). In general, an Aβ peptide can be between Aβ1-39 and Aβ1-43 (e.g., Aβ1-40 or Aβ1-42), Aβ(12-24), Aβ12-28, or Aβ(14-23). In general, an Aβ peptide used in an *in vitro* assay is an Aβ peptide that forms β-form and subsequently forms fibrils, e.g., Aβ(1-42) (e.g., Selkoe, 2000, JAMA 283:1615-1617).

A "discordant helix" is an amino acid sequence that is able to form an α-helix and is also predicted to be able to form a β-strand. A discordant helix can be identified using structure analysis programs that predict secondary structure of polypeptides, specifically by analyzing an amino acid sequence for experimentally determined (for example, by NMR or crystallography) α-helix and also analyzing the amino acid sequence for predicted β-strand. A sequence that is experimentally determined to form α-helix and is predicted to form a β-strand is a discordant helix. A discordant helix amino acid sequence can be an isolated peptide, or form part of a polypeptide. A discordant-helix can be naturally occurring in a wild type or mutant polypeptide. A discordant helix can also be in a synthetic amino acid sequence. In general, the discordant helix amino acid sequence is at least about 6 amino acids in length. Such sequences can be longer, e.g., 7, 8, 9, 10, 11, 12, 14, 16, 18, 22, 24, or 26 amino acids in length. A discordant helix can also be determined using other methods that can identify a sequence that is both predicted to form or is experimentally shown to form α-helix and is predicted to form or is experimentally shown to form β-strand.

A "polypeptide" means a chain of amino acids regardless of length or post-translational modifications.

A "non-amyloidogenic form" of a polypeptide containing a predicted discordant helix is the form of the protein in which α-helix is the predominant conformation of the discordant amino acid sequence. Compounds that promote the α-helix conformation of a discordant helix are useful for preventing the formation of amyloid. A non-amyloidogeric form can be a form of a discordant sequence that is predicted to be in an α-helical conformation with higher frequency than a corresponding sequence (e.g., an allele).

A disorder related to Alzheimer's disease is a disorder in which peptides derived from the APP protein have been demonstrated to be present or are suspected of being present. Such disorders include dementia pugilistica, Down syndrome, and severe head trauma.

Unless otherwise defined, all technical and scientific terms used herein have the same meaning as commonly understood by one of ordinary skill in the art to which this invention belongs.

The materials, methods, and examples are illustrative only.

### DESCRIPTION OF DRAWINGS

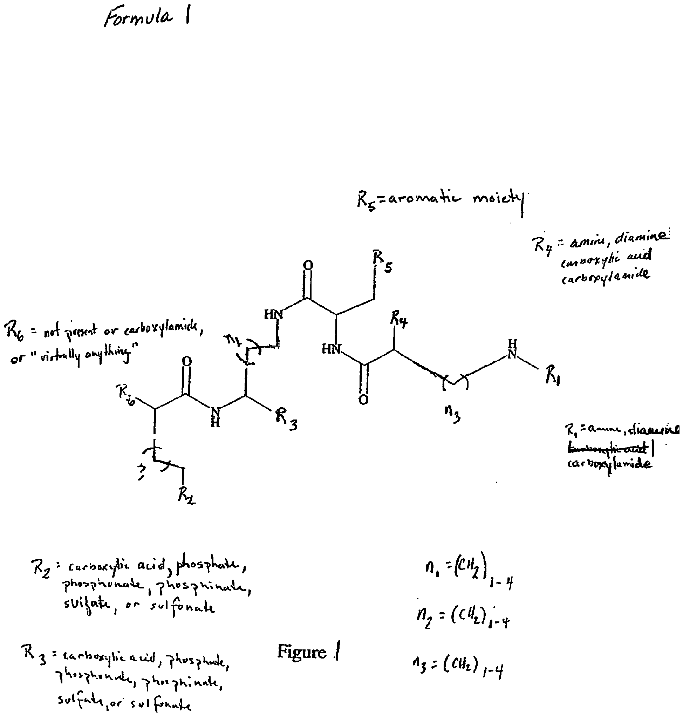

Fig. 1 is a depiction of the chemical structure of Formula 1 (ligand 1).

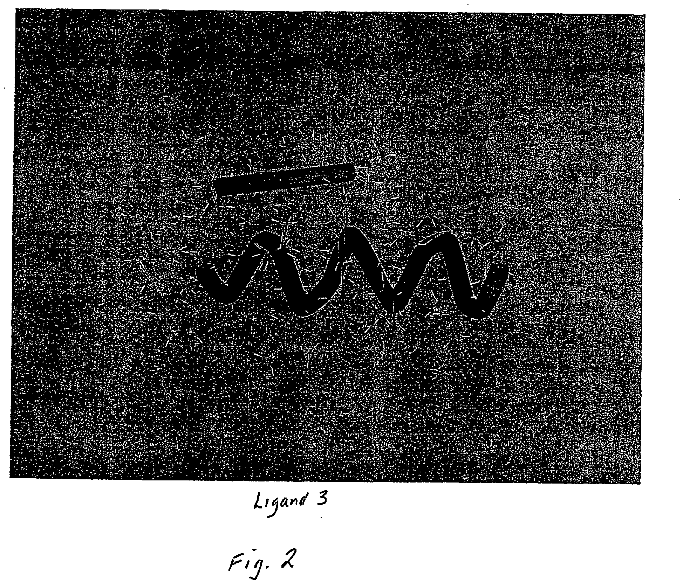

Fig. 2 is a depiction of the structure of a complex of ligand 3 (depicted by a light cylinder in the upper part of the figure) and Aβ(12-28) (depicted by a dark cylinder in the lower part of the figure) after 20 ps of molecular dynamics simulation, starting from all-helical structure of Aβ(12-28).

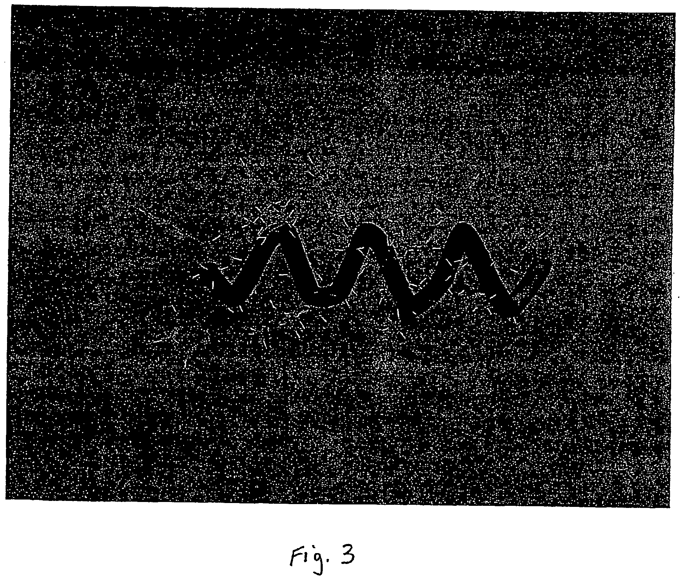

Fig. 3 is a depiction of the completely helical structure of Aβ(12-28) before molecular dynamics simulations.

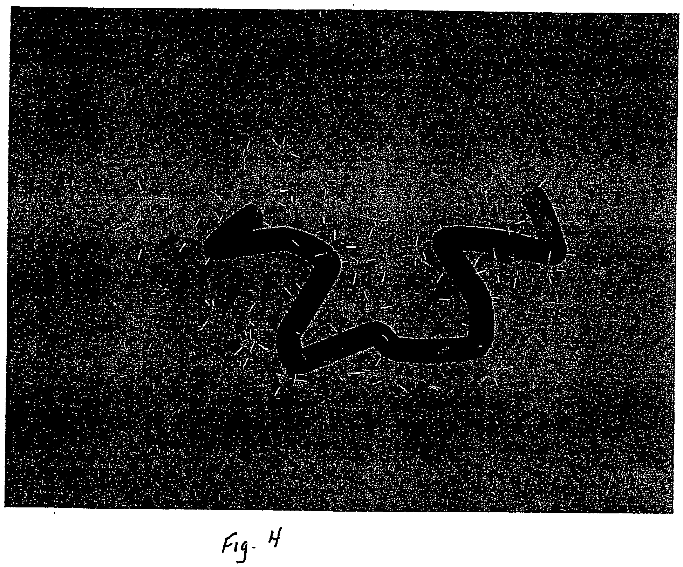

Fig. 4 is a depiction of the structure of Aβ(12-28) after 20 ps of molecular dynamics simulation in the absence of ligand, starting from a completely helical structure.

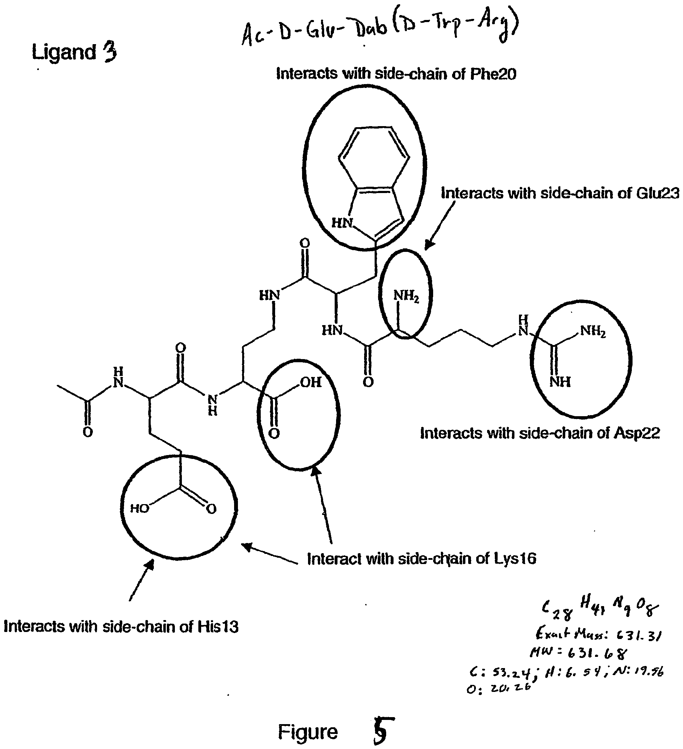

Fig. 5 is a depiction of the chemical structure of ligand 3 and identification of the side-chains of Aβ amino acid residues that interact with the ligand, provided that the corresponding portion of Aβ forms an α-helix.

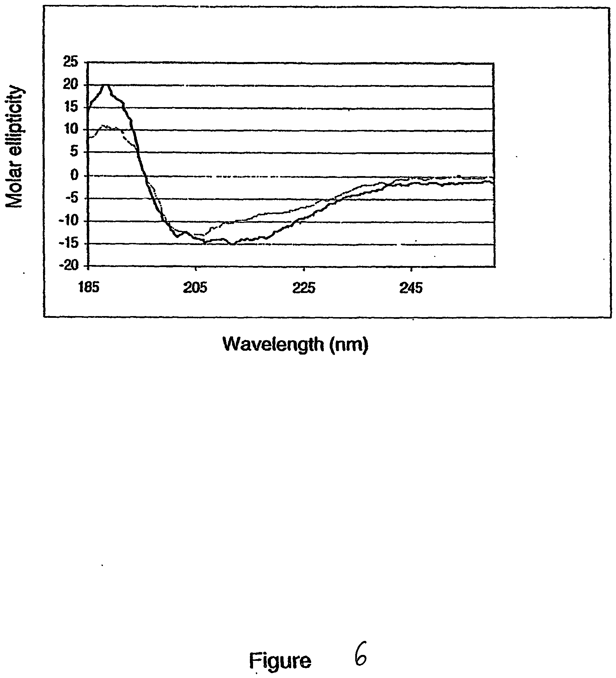

Fig. 6 is a graph illustrating the results of a circular dichroism spectra analysis of Aβ(12-28) (light line) and Aβ(12-28) mixed with a ligand of Formula 1 (ligand 3) in a 1:1 molar ratio (dark line) (the spectrum of the Aβ/ligand mixture is the one with the lowest value at 222 nm and highest value at 195 nm), in sodium phosphate buffer, pH 7, containing 30% (v/v) trifluoroethanol, 20°C. The spectrum of the ligand was subtracted from the spectrum of the Aβ-ligand mixture.

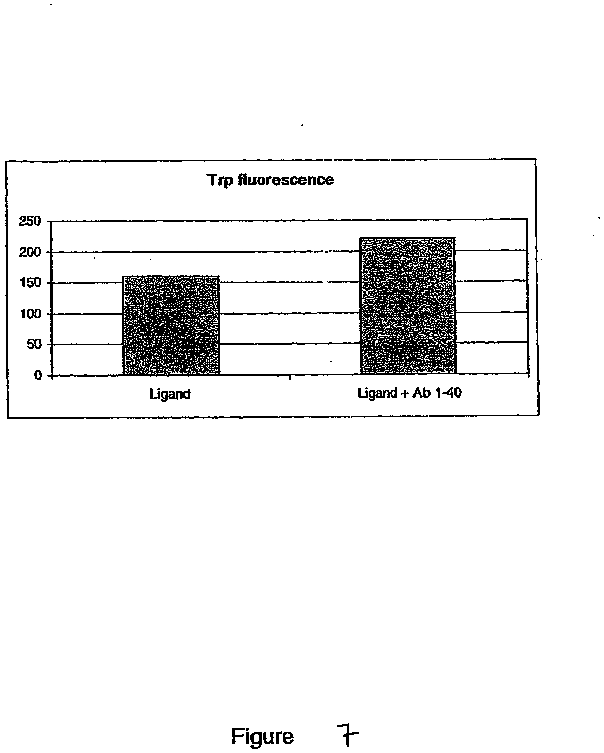

Fig. 7 is a bar graph illustrating the results of a tryptophan fluorescence emission analysis of a ligand of Formula 1 (ligand 3) (left column) and Aβ(1-40) mixed with the ligand (right column) in a 1:1 molar ratio, in sodium phosphate buffer, pH 7, containing 30% (v/v) trifluoroethanol. The emission maximum was at 348 nm for the ligand only, and at 344 nm for the complex. Excitation was at 280 nm, 20°C.

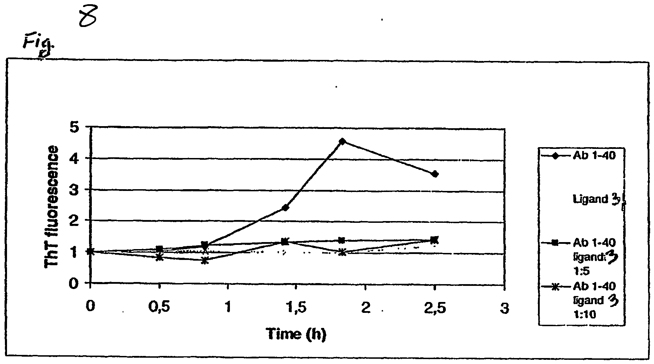

Fig. 8 is graph depicting the results of experiments in which thioflavin T fluorescence of Aβ(1-40) was assayed after different periods of incubation at 37°C in 10mM sodium phosphate buffer, pH 7, and the thioflavin T fluorescence of Aβ(1-40) mixed with ligand 3 at 5:1 or 10:1 ligand/Aβ molar ratios. The thioflavin T fluorescence of the ligands only is also shown. The values are the mean of duplicate samples.

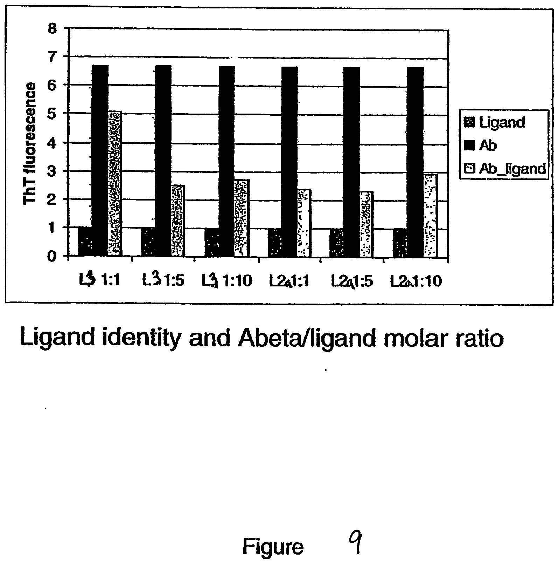

Fig. 9 is a bar graph depicting the results of experiments in which the thioflavin T fluorescence of Aβ(1-40) was detected after 1.5 hours of incubation at 37°C in 10mM sodium phosphate buffer, pH 7, and the thioflavin T fluorescence of Aβ(1-40) mixed with ligand 3, or ligand 2a at 1:1, 5:1, or 10:1 ligand/Aβ molar ratios. The thioflavin T fluorescence of the ligands only is also shown. The values are the mean of duplicate samples.

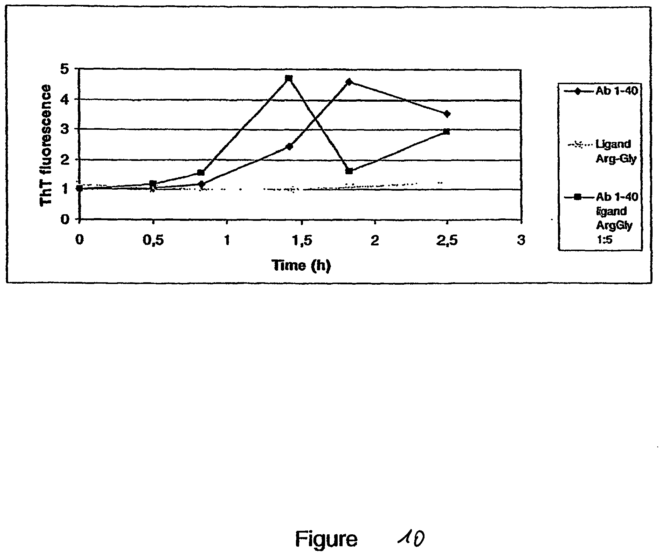

Fig. 10 is a graph depicting the results of experiments in which thioflavin T fluorescence of Aβ(1-40) was detected after different periods of incubation at 37°C in 10mM sodium phosphate buffer, pH 7, and the thioflavin T fluorescence of Aβ(1-40) mixed with the dipeptide Arg-Gly at 5:1 ligand/Aβ molar ratio. The thioflavin T fluorescence of the ligand only is also shown. The values are the mean of duplicate samples.

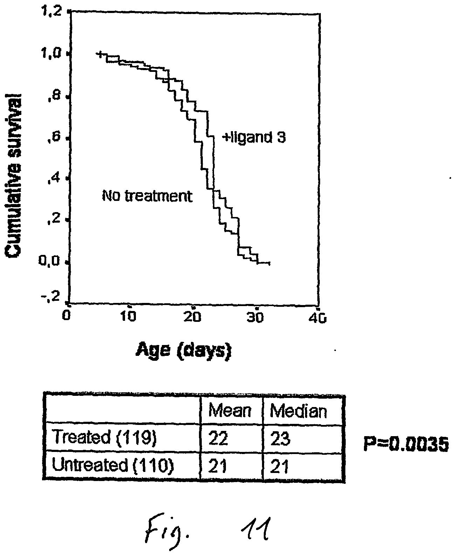

Fig. 11 is a graphic representation of the results of experiments in which the number of living flies was determined after eclosion for *Drosophila* that express Aβ(1-42) without (black line) or with treatment with 650 µM ligand 3 in the food (+ ligand 3, grey line). The plotted data are the number of living flies in the respective group at the indicated number of days after eclosion. Also depicted are the mean and median survival times in days for treated and untreated flies (p=0.0035).

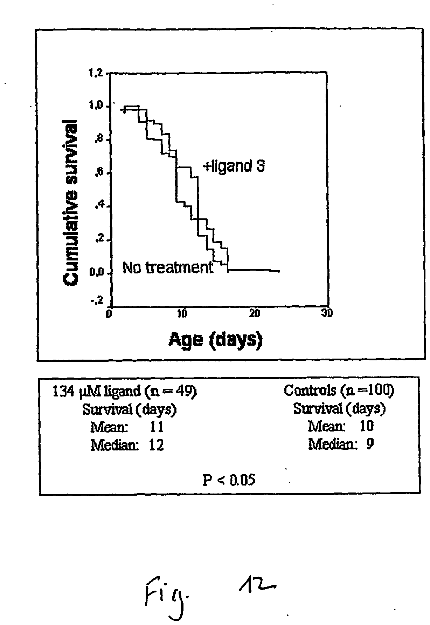

Fig. 12 is a graphic representation of the results of experiments in which the number of living flies was determined after eclosion for *Drosophila* that express Aβ(1-42) without (no treatment, black line) or with treatment (+ ligand 3, grey line) with 134 µM of ligand 3 in the food. Also depicted are the mean and median survival times in days for treated and untreated flies in these experiments.

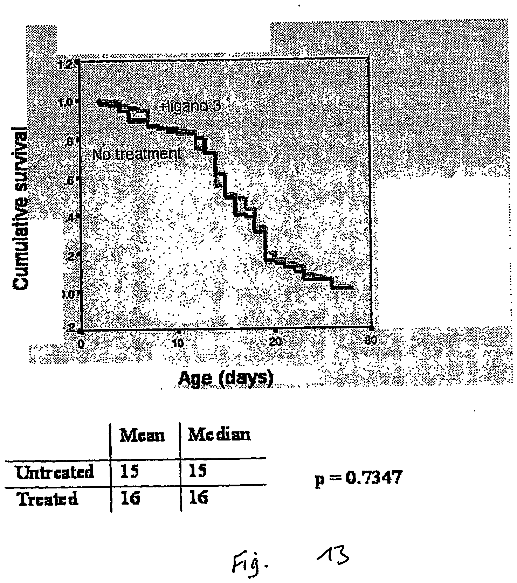

Fig. 13 is a graphic representation of the results of experiments in which the number of living flies was determined after eclosion for wild type *Drosophila* (WIII8) that do not express Aβ(1-42) without (no treatment, grey line) or with treatment with 650 µM ligand 3 in the food (+ ligand 3, black line).

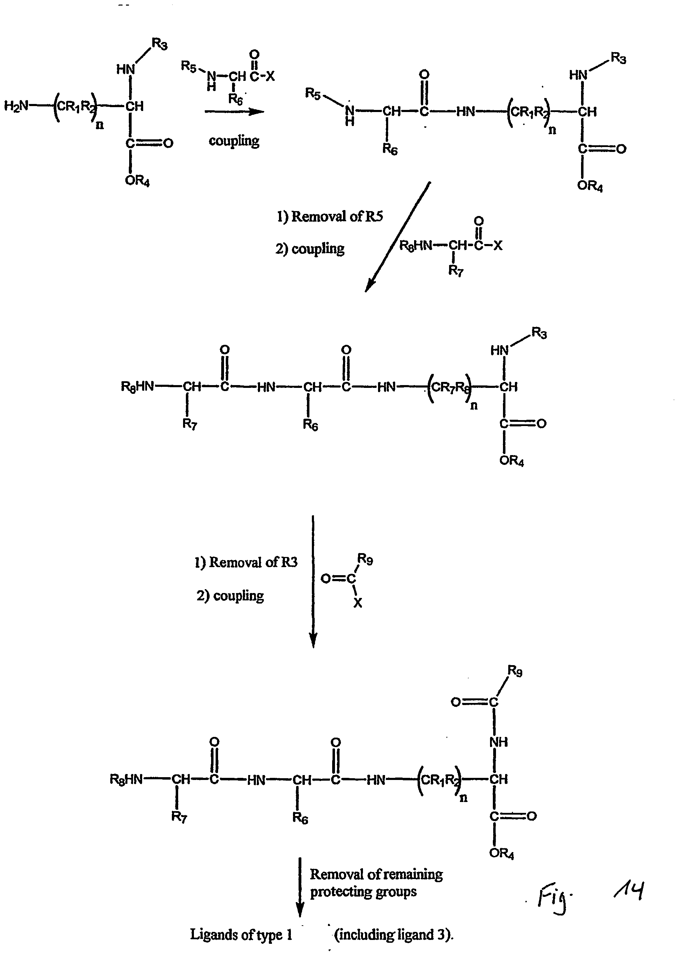

Figure 14 is a schematic drawing depicting a general scheme for synthesizing compounds of Formula 1.

### DETAILED DESCRIPTION

Stabilization of an Aβ discordant helix can reduce the amount of β conformation peptide and so reduce the amount of amyloid formed. Compounds that can bind to the Aβ discordant helix were designed using molecular modelling. The side-chains of residues Lys16, Phe20, Asp22, Glu23, and His13 of Aβ were targeted by the designed ligands. In brief molecular dynamics simulations binding of any of the ligands to Aβ in a 1:1 complex delays or prevents the unfolding of the Aβ discordant helix. This interaction was experimentally verified in several ways. The addition of the ligands to Aβ, in ligand/Aβ molar ratios ranging between 1 and 10, was found to result in (i) formation of an Aβ-ligand complex as verified by changes in tryptophan fluorescence of the ligands (Fig. 12), (ii) increased helical content of Aβ as studied by circular dichroism spectroscopy, and (iii) reduced fibril formation of Aβ, as studied by thioflavin T fluorescence build-up. Increasing the α-helical content of Aβ by addition of trifluoroethanol resulted in increased Aβ-ligand binding, thereby demonstrating that the ligands work as predicted from their design. Thus, stabilization of the Aβ discordant helix can be achieved by presenting Aβ with helix-binding ligands, which can result in reduced fibril formation (fAβ). Such compounds are useful for reducing Aβ aggregation and fibril formation *in vivo*, and therefore such compounds are candidates for treatment of conditions in which it is desirable to decrease aggregation of Aβ, in particular, in Alzheimer's disease (AD) and related disorders. In general, a compound as described herein can stabilize an Aβ peptide in an α-helical conformation. A stabilized Aβ peptide has an increased probability of being in an α-helical conformation compared to a peptide in the absence of ligand.

Therefore, in some aspects, the invention relates to methods of designing and identifying compound that stabilize Aβ peptide structure, e.g., candidate compounds for treating disorders associated with the accumulation of fAβ, e.g., Alzheimer's disease. It also relates to compounds for treating or preventing such disorders in a subject suffering from the disorder or at risk for having the disorder.

The invention provides compounds of Formula 1, 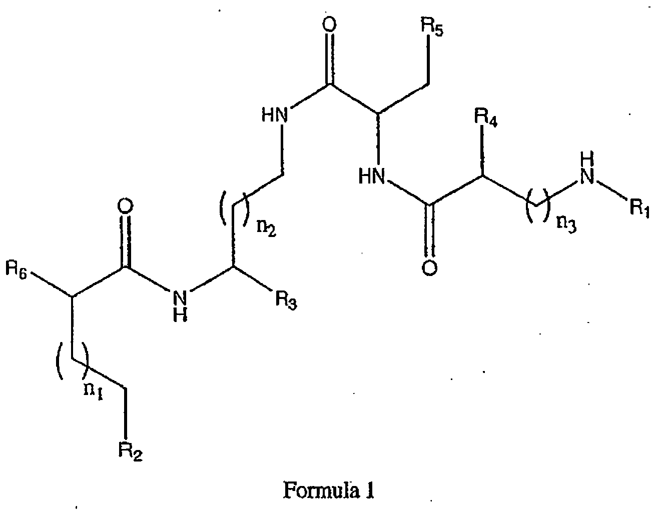 and pharmaceutically acceptable salts and hydrates thereof, wherein:
n₁ is (CH₂)₁₋₄;
n₂ is (CH₂)₁₋₄;
n₃ is (CH₂)₁₋₄;
R₁ is an amine, diamine, -C=NH(NH₂), or carboxylamide
R₂ is carboxylic acid, phosphate, phosphonate, phosphinate, sulfate, or sulfonate; R₃ is carboxylic acid, phosphate, phosphonate, phosphinate, sulfate, or sulfonate; R₄ is an amine, diamine, carboxylic acid, or carboxylamide;
R₅ is an aromatic ring moiety; and
R₆ is not present or is a carboxylamide or any other group that does not interfere with the association of the compound with the Aβ peptide.

The following are examples of suitable R groups.

In one embodiment, R₁ is -C=N(NH2).

In one embodiment, R₂ is -C(O)OH.

In one embodiment, R₃ is -C(O)OH.

In one embodiment, R₄ is an amine.

In one embodiment, R₅ is an aromatic ring associated with tryptophan.

In one embodiment, R₆ is -NHC(O)CH₃.

A general scheme for synthesis of compounds described herein such as compounds of Formula 1 is shown in Fig. 14. Such syntheses are generally performed using peptide and general amide synthesis methods for assembly and protection/deprotection. "Couplings" can be effected using any of numerous methods and condensing agents that are generally known in the art. The order of assembly of the building blocks can also be varied.

Amide linkages that are depicted in the scheme can also be replaced by ester linkages and N-alkylated amide linkages and synthesized using methods similar to those described herein. Building blocks with additional methylene or substituted methine groups on either side of the depicted alpha CH groups can be assembled using similar technologies. It is also possible to perform the synthesis when the H of the depicted alpha CH groups are replaced by alkyl or substituted alkyl groups.

Additional guidance for synthesizing compounds is provided *in* the Examples.

### Identification of Compounds that Stabilize α-Helical Conformation

The invention includes methods of screening designed compounds for at least one of the following indicia indicating that the compound is useful for treating AD; the ability to stabilize or increase the amount of an α-helical form of an Aβ peptide, to decrease the amount of a β *form of* an Aβ peptide, to slow the accumulation of an β form of an Aβ peptide, inhibit the amount of fibril formation of an Aβ peptide, or the effect of the compound on the amount of discordant helix in a sample of Aβ peptide. Additional indicia include a decrease in the rate of accumulation or the total amount of Aβ fibrils or amyloid plaques in an animal that is susceptible to AD-like pathology or is susceptible to forming such structures containing Aβ. In addition, an animal or human can be assayed for a decrease in the rate of accumulation of symptoms of AD or a related disorder (e.g., a decrease in the rate of memory loss). A decrease in the rate of accumulation of symptoms in an animal or human treated with a compound as described herein indicates that the compound is useful for treatment of AD or a related disorder.

The amount of Aβ discordant helix can be determined by measuring the amount of α-helix in a sample containing an Aβ polypeptide in the presence and absence of the particular compound. Any suitable method that detects the presence of α-helix or alternatively, β-structure, can be used to screen test compounds for their ability to stabilize an α-helix of an Aβ discordant helix. Such methods include NMR (e.g., Johansson et al., 1994, Biochemistry 33:6015-6023), circular dichroism (CD) (Johansson et al., 1995, FEBS Lett. 362:261-265; Wang, 1996, Biochim. Biophys Acta 1301:174-184), and Fourier transform infrared spectroscopy (FTIR; Vandenbussche, 1992, Biochemistry 31:9169-9176). α-Helix stability can be assessed from the rate of decrease in the amount of the soluble, α-helical form of the peptide/protein (e.g., the half-life of the α-helical form) in the presence and absence of a test compound, e.g., using electrospray (ES)-mass spectroscopy. ES or matrix-assisted laser desorption/ionisation (MALDI) mass spectrometry in combination with hydrogen to deuterium (H/D) exchange mass spectroscopy can also be used to assay for the presence and/or stability of an α-helix conformation of a discordant helix. In this approach, the kinetics of H/D exchange of the soluble forms of a discordant helix-containing polypeptide are studied.

H/D exchange rates at specific residues can be determined by NMR. Such NMR analysis generally requires pure samples at high concentration, and long time periods for measurements, which make analysis of some polypeptides difficult. These problems can be partially solved by the application of mass spectrometry. Using either ES or MALDI mass spectrometry, H/D exchange levels can be monitored at low concentrations. The analysis times are short and mixtures of peptides can be analyzed. Thus, mass spectrometry is a particularly useful technique for studying the relative H/D exchange rates of protein mixtures (Hosia et al., 2002, Mol. Cell. Proteomics 1(8):592-597).

Mass spectrometry is a technique that can be used to investigate non-covalent interactions involving proteins such as those involved in the formation of α-helices. To study non-covalent interactions directly using mass spectrometry, it is important that the protein be, to the extent possible, in its non-denatured state. This can be accomplished using ES as the method of ionization for the mass spectroscopy analysis. ES involves spraying the protein that is in an aqueous solution at physiological pH in the absence of an organic co-solvent (Pramanik et al., 1998, J. Mass Spectrom. 33:911-920). An additional requirement for the use of ES in analysis of proteins in their native states is the careful control of the de-clustering voltage (cone voltage) within the ES interface. An excessive potential difference can lead to collision-induced dissociation or the destruction of non-covalent associations.

### ES-mass Spectrometry

Mass spectrometry can be used in combination with H/D (hydrogen/deuterium) exchange to obtain information concerning non-covalent interaction and tertiary structure and stability (Smith et al., 1997, J. Mass Spectrom. 32:135-146; Mandel et al., 1998, Proc. Natl. Acad. Sci. USA 95:14705-14710). In ES-mass spectroscopy, spectra can be recorded using a quadrupole-time-of-flight (TOF) instrument (Micromass, Manchester, England). The instrument can be fitted with an orthogonal sampling nano-ES interface (Z-Spray) consisting of a quadrupole mass filter, a hexapole collision cell and an orthogonally arranged TOF analyzer complete with reflectron (Morris, 1996, Rapid Commun. Mass Spectrom. 10:889-896). Samples can be sprayed from gold-coated borosilicate capillaries (Protana A/S, Denmark). A suitable collision gas such as argon (AGA, Sweden) is used for collision-induced dissociation (CID). To determine the relative rate of disappearance from solution of various forms of an Aβ peptide polypeptide (e.g., the disappearance of the α-helical form), an appropriate acquisition range is selected, for example, m/z 135-4000. Three hundred scans of five seconds duration are recorded for each time point and combined into one spectrum. The ion currents corresponding to the summed abundance of singly and multiply, e.g., doubly and triply protonated molecules, can be determined using maximum entropy software (Micromass). CID spectra can be recorded to confirm the structure of the polypeptide. In H/D exchange, the rates of H/D exchange reactions are monitored by continually recording ES mass spectra. At specific time points, the ions corresponding to the H/D exchanged forms of the protein of interest are subjected to CID. The location of the exchanged protons is indicated by the resultant fragmentation spectrum.

### MALDI Mass Spectra

MALDI mass spectra can be recorded, e.g., on a Voyager-DE.TM. PRO Biospectrometry Workstation (PerSeptive Biosystems Inc.) operated in the positive ion mode. Deuterated or non-deuterated polypeptide is dried on a surface (e.g., a 100 well plate) containing pre-dried α-cyano-4-hydroxycinnamic acid (20 µg), which for the recording of H/D exchange spectra is applied from a solution in CH₃CN/D₂O. The instrument is equipped with a 335 nm laser and operated in the linear mode employing delayed extraction. An appropriate acquisition range is used (e.g., m/z 3500-4000) and external calibration is employed (e.g., between m/z 3660.19 and 5734.58). A spectrum is calculated as an average of a number of acquisitions (e.g., 400 acquisitions), and triplicate samples are generally recorded for each time point. The number of protons exchanged with deuterons at each time point is calculated by subtracting the masses of the peptides in the protonated solvent from the masses of the peptides in the deuterated solvent. This is done, e.g., to correct for ongoing intramolecular disulfide formation. Such intramolecular disulfide formation may be observed by ES CID. In experiments to compare the rate of H/D exchange as measured by MALDI combined with ES, identical solutions are spotted on the MALDI plate and sprayed from the ES capillary.

### Determination of Rate Constants from Decay Curves

Estimation of rate constants from mass spectrometric data can be performed using, for example, non-linear least squares regression in Matlab.RTM version 5.3 (Mathworks, Natick, Mass., USA) using the routines in the Mathworks Optimization Toolbox. Absolute concentrations are not available from the data, but this is not required as the helix unfolding is a first-order reaction (e.g., Szyperski, 1998, Protein Sci. 7:2533-2540). Instead, ion counts can be used as the concentration unit in the non-linear regression, assuming that this measure is proportional to concentration for each peptide. Marginal standard deviations for the rate constants can be calculated by the standard procedure of linearization of the objective function (Seber et al., 1988, Non-linear Regression, John Wiley & Sons, New York)

### Assay of Test Compounds

The compounds described herein have been or can be assayed for their ability to stabilize an α-helical form of an Aβ peptide that contains a discordant helix. The methods described herein can be used for such assays. Typically, the test compound is added to a solution containing the polypeptide, and the content of helical structure or rate at which the α-helix form of the peptide disappears from the solution is determined, e.g., using CD, FTIR, NMR spectroscopy, ES-mass spectroscopy, or MALDI mass spectrometry. The effects of test compounds on fibril formation can also be determined as described *infra.* The helical content and/or the rate of disappearance of α-helix in the presence and absence of the test compound is determined. An increased helical content and/or a decreased rate of α-helix disappearance from the solution in the presence of the test compound indicates that the test compound is a candidate compound useful for treating AD or a related disorder.

Alternatively, after incubation of a solution containing discordant helix-containing an Aβ peptide in the presence of a test compound for a fixed time, the amount of α-helix can be determined and compared to the amount of α-helix present in the polypeptide incubated for the same amount of time in the absence of the test compound. A larger amount of α-helical form of the polypeptide in the solution containing the test compound compared to the solution that did not contain the test compound indicates that the test compound stabilizes the α-helical form of the peptide (i.e., is a candidate compound).

Appropriate incubation times can be determined empirically. The incubation times can be minutes, hours, days, weeks, or months. For example, the incubation time for an Aβ(140) at a concentration of about 100 µM can generally form fibrils *in vitro* that are detectable by electron microscopy within three days. Other aggregates can form more rapidly (minutes to hours). One skilled in the art will understand how to determine an appropriate incubation time, depending on the Aβ peptide used and the method of detection that is used. Various parameters can be manipulated to modulate the rate of α-structure formation, e.g., by increasing concentration of the polypeptide or increasing the salt concentration in the solution.

### Test Compounds and Candidate Compounds

Test compounds are compounds that are screened for their ability to increase the helical content of a solution containing Aβ peptides and/or slow the rate of conversion of the α-helix form of an Aβ peptide to a β-strand structure. Candidate compounds are compounds found to stabilize the α-helical form of an Aβ peptide. Candidate compounds are useful for preventing or slowing the formation of β-strand structures and amyloid.

Methods are provided herein for confirming the ability of a compound, as described herein, to increase the stability or formation of the α-helix conformation of an Aβ peptide. In general, compounds are designed to have a surface that is complementary to a surface of an Aβ peptide discordant helix (e.g., the two surfaces are capable of an electrostatic interaction), will bind to that surface, and can stabilize the helix. This interaction is predicted to reduce the number of events in which the discordant helix converts to a β-strand conformation, thereby reducing the amount of fibril formation. Interactions between such compounds and a discordant helix can be non-covalent. The compounds include those compounds described in the Examples. The compounds used in the screening assays can be dipolar, neutral, or mono-charged.

### Additional Methods of Assaying Test Compounds

### Direct Detection of Fibril Formation

In some cases methods other than those that assay stabilization of α-helix conformation can be useful for the invention. For example, direct detection of fibril formation can be used instead of or in combination with an assay that measures α-helix conformation. The extent and rate of fibril formation in the presence or absence of a test compound can be measured directly by electron microscopy (EM) of fibrils formed. For example, pellets containing fibrils are suspended in a small volume of water using low energy sonication for five seconds. Aliquots of the fibril suspension are then placed on grids covered by a carbon-stabilized Formvar™ film. Excess fluid is withdrawn from the grids after 30 seconds. The grids are then air-dried and negatively stained with 3% uranyl acetate in water. The stained grids are then examined and photographed in an electron microscope such as a Phillips CM120TWIN operated at 80 kV. Compounds that result in a reduced rate or amount of fibril formation are useful for the invention, e.g., are candidate compounds for treating AD or a related disorder.

Fibril formation can also be assessed by staining the fibrils with dyes, e.g., Congo red or Thioflavin T, and detecting emitted light from the stained sample. For example, pellets containing fibrils are resuspended in phosphate buffered saline by sonication before addition of Congo Red (2% w/v). The samples are incubated for one hour at ambient temperature and aggregated proteins collected by centrifugation at 13,400 x g for five minutes. The aggregates are then washed in water and resuspended in water. An aliquot is placed on a microscope slide, dried, and observed by polarization microscopy (e.g., using a Zeiss Axialfold microscope). Alternatively, detection of light absorption or emission at different wavelengths after staining with Congo red or Thioflavin T can be used to quantify amyloid formation (e.g., LeVine, 1993, Prot. Science 2:404-410; Klunk et al., 1999, Anal. Biochem. 266:266:66-76).

An assay for detection of fibril formation in the presence and absence of a test compound can be used, e.g., to prescreen test compounds for those that are to be used in subsequent assays of α-helix stabilization. Similarly, the ability of a compound to inhibit fibril formation can be used to confirm the predicted efficacy of a candidate compound in preventing fibril formation.

### Indirect Detection of Fibril Formation

Fibril formation can also be indirectly assayed by measuring the disappearance of the α-helical forms that, after α-helix to β-strand conversion and aggregation, give rise to fibrils. For this purpose, ES mass spectrometry is a method that can be used since it can be used to distinguish between monomeric and aggregated forms of an Aβ peptide that contains a discordant helix. Alternatively, aggregates can be removed, e.g., by centrifugation, and peptides remaining in solution (which are predominantly α -helical in their discordant helix region) are then quantified by techniques such as gel electrophoresis, amino acid analysis, or reversed-phase HPLC.

As with direct methods of assaying fibril formation, the indirect methods are useful for identifying compounds that interfere with α-helix to β-strand conversion and therefore will inhibit amyloid fibril formation, e.g., for screening test compounds to be used in assays for stabilization of an α-helix conformation of a discordant helix-containing polypeptide or to confirm that a candidate compound has a stabilizing effect.

The effect of different compounds on fibril formation can be observed using the above methods, preferably in combination as they have complementary profiles. Thus, staining techniques are fairly rapid, allowing screening of many compounds. Electron microscopy (EM) is more specific than dye detection since with EM the nature of the fibrils can be judged and fibrils can be quantitatively analyzed. Mass spectrometry is highly sensitive, while gel electrophoresis, amino acid analysis and reversed-phase HPLC can be used with a large number of samples but are less sensitive.

Thus, in screens to confirm the ability of a compound to be useful as described herein, several stages of assay can be used. For example, a compound can be tested using staining methods, and also tested with ES mass spectroscopy, e.g., to confirm the ability of a compound to inhibit the amount of β-structure in an Aβ peptide.

When characterizing the effects of different compounds, both the amount of fibril formed and the rates of fibril formation can be of interest, since comparatively minor changes in the rates of fibril formation *in vitro* may reflect the tendency of a peptide/protein to form amyloid fibrils over a long time span *in vivo.*

### Identification of Compounds that Reduce Aβ Aggregation and Fibril Formation

Experimental data and theoretical models indicate that α-helix to β-strand conversion of the 16-23 region of Aβ is critical for amyloid fibril formation. Since Aβ positions 16-23 show helical structures in the presence of membrane-mimicking solvents or detergents, it is likely that this region is also helical in membrane-associated APP. However, in liberated Aβ in an aqueous solution, a helical conformation will be only transiently stable, in line with the unordered Aβ conformation in water detected by spectroscopic methods (Serpell, 2000, Biochim. Biophys. Acta 1502:16-30). Conversion of the 16-23 region into extended/β-strand conformation is required for formation of fibrils built up from β-sheets.

### Ligands for Stabilizing Discordant Helices Associated with Aβ

Fig. 1 illustrates the general structure of Formula 1 (ligand 1), in which n1 can be (CH₂)₁₋₄; n2 can be (CH₂)₁₋₄; n3 can be (CH₂)₁₋₄; R1 can be an amine, diamine, carboxylic acid, or carboxylamide; R2 can be carboxylic acid, phosphate, phosphonate, phosphinate, sulfate, or sulfonate; R3 can be carboxylic acid, phosphate, phosphonate, phosphinate, sulfate, or sulfonate, R4 can be an amine, diamine, carboxylic acid, or carboxylamide; R5 can be an aromatic ring associated with tyrosine, phenylalanine, or tryptophan; and R6 is not present or is a carboxylamide or any other group that does not interfere with the association of the compound with the Aβ peptide. One skilled in the art will understand how to select such a group based on general knowledge of the art and the examples of ligands provided herein.

In one example, ligand 3 (Fig. 2 and Fig. 5), n1 is CH₂; n2 is CH₂; n3 is (CH₂)₃; R1 is a diamine; R2 is a carboxylic acid; R3 is a carboxylic acid, R4 is an amine; R5 is an aromatic ring associated with tryptophan; and R6 is a carboxylamide.

For all ligands described herein, the chiral carbon atoms can be either stereoisomer, and a composition may contain mixed stereoisomers. A composition can also contain more than one type of ligand including containing more than one variant of a ligand.

The ligands are further described and illustrated in the Examples and the Drawings.

### In Vivo Testing of Compounds

Animal models of Alzheimer' disease or other amyloidoses can be used to further test compounds disclosed herein. The animals used can either represent naturally occurring models (e.g., animals that naturally accumulate amyloid such as non-human primates including, without limitation *Macaca mulatta*), animals that are genetically engineered to express an amyloid peptide, or animals that are treated to induce the production of amyloid. In general, genetically engineered animal models are used to determine the efficacy of a compound for reducing the deposition of an Aβ peptide. Reducing the deposition of an Aβ peptide can include, without limitation, a reduction in the amount of Aβ-containing structures (e.g., fibrils (soluble or insoluble) or amyloid plaques) detected in the animal model, slower accumulation of Aβ-containing fibrils in the presence of the compound compared to an animal that was not treated with the compound, a reduction of symptoms associated with the deposition of amyloid, or a slowing of the acquisition of symptoms associated with the deposition of amyloid. Such models and methods of detecting Aβ and amyloid structures are known in the art.

Invertebrate models can also be used for testing a compound, e.g., *Drosophila melanogaster.* Genetically engineered *Drosophila* can be useful for identifying compounds that are effective *in vivo* for inhibiting formation of amyloid-related structures. For example, transgenic expression of Aβ(1-40) and/or Aβ(1-42) linked to a signal peptide that is unrelated to the naturally occurring APP protein from which the peptide is naturally derived can be used. The signal peptide is used to direct the Aβ expression to the endoplasmic reticulum and subsequent secretion (as for the APP) in *Drosophila* eyes or nervous system (Finelli et al., 2004, Mol. Cell. Neurosci. 26:365-375; Crowther et al., 2004, Curr. Opin. Pharmacol. 4:513-516). In such models, expression of multiple copies of Aβ(1-42), but not Aβ(1-40), generally results in Aβ aggregation and neuronal degeneration. A *Drosophila* model that recapitulates the expression and processing of human APP, which results in generation and toxicity of Aβ, has also been described (Greeve et al., 2004, J. Neurosci. 24:3899-3906) and can be used. For example, a *Drosophila* strain that is genetically engineered to contain two copies of a sequence encoding human Aβ(1-42) and to express those peptides in the animals' nervous systems can be used. The Aβ coding sequence is operatively linked to a signal peptide (e.g., the *Drosophila* necrotic gene; Green et al, 2000, Genetics 156:1117-1127). Expression of the peptide in the animals results in Aβ aggregation, neuronal degeneration, reduced life-span, and decreased locomotor activity (Crowther et al., 2004, In: Proceedings of the 45th Annual Drosophila Research Conference; Washington DC, USA. The Genetics Society of America; 2004:95; Crowther et al., 2005, Neuroscience 132:123-135). To test a compound, the fly larvae are fed a compound by mixing it into their food (about 0.1-1.5 mg/ml ligand). Controls are fed on food containing only the solvent in which the ligand is dissolved or a control compound (e.g., in the solvent) that is not predicted to slow the loss of α-helix (at concentrations of between about 0.1 mg/ml and 100 mg/ml). Larvae or adult flies are examined for one or more features associated with Aβ. A decrease in one or more such features in animals receiving the compound indicates that the compound is useful for treating a disorder associated with an Aβ peptide.

Transgenic mice expressing human Aβ peptides can be used to test compounds for their ability to prevent or decrease the amount of amyloid. In additional behavioural tests can be used to determine whether a compound reduces or prevents the development of behavioural stigmata associated with the expression of an Aβ peptide in such mice. In one murine model system, an amyloid precursor protein (APP) gene having a mutation associated with AD (e.g., early onset AD) is expressed. The expression of such APP proteins generally increases the production of Aβ. One example of such a gene bearing an APP mutation is the "Swedish" mutation gene sequence, K670N/M671L. Another mutation that increases production of Aβ and/or increases the production of the more amyloidogenic Aβ(1-42) relative to Aβ(1-40) (e.g., the "London" mutation, V717I) (Hutter-Paier et al., 2004, Neuron 44:227-238). In addition to APP, transgenic expression of mutant (e.g., A246E) presenilin1 (PS1), which also increases the production of the more amyloidogenic Aβ(1-42) relative to Aβ(1-40), is used (e.g., Permanne et al., 2002, FASEB J. 16:860-862). Transgenic expression of other mutants of PS 1, combined with mutant APP expression, has been described (Casas et al., 2004, Am. J. Pathol. 165:1289-1300). In addition to transgenic APP and PS 1 expression, the tau protein, which becomes hyperphosphorylated and forms neurofibrillary tangles in AD, can be expressed, resulting in a triple transgenic mouse which reproduces several of the pathophysiological features seen in AD patients (Oddo et al., 2003, Neurobiol. Aging 24:1063-1070).

In one example of an animal model used for *in vivo* evaluation of a compound for treatment of AD, wild-type rats are used in which fibrils are formed *in vitro* from amyloid β-peptide (Aβ) that is injected into the brain parenchyma (e.g., Chacon et al., 2004, Mol. Psychiatry 9:953-961; Soto et al., 1998, Nature Med. 4:822-826). The effect of a compound as described herein on the brains of such animals can then be evaluated.

### Drug Administration

A compound as described herein can be administered using different methods. In some cases, different methods of administration are evaluated for their efficacy and/or the half-life of the compound in the animal, e.g., in peripheral blood or cerebrospinal fluid (CSF).

In mice, oral administration, intraperitoneal injection, intracerebral injection (e.g., into the hippocampus), and intra-cerebroventricular infusion can be used. The total doses and dosing schemes can be varied to identify an effective dose. In such testing, compounds are generally administered in doses in milligram amounts, e.g., total doses of about 1-100 mg, 1-50 mg, or 50-100 mg are administered three to four days per week for several weeks to achieve the total dose, (e.g., Permanne et al., 2002, FASEB J. 16:860-862). For *Drosophila,* as described above, oral administration is generally used (i.e., the compound is mixed with the food), however, injection and transcutaneous administration can be used. In general, compounds described herein are administered in doses from about 1-500 mg, 1-300 mg, 100-500 mg, 100-300 mg, 1-100 mg, 1-50 mg, 1-30 mg, 10-50 mg, or 10-20 mg. For example, oral administration of ligand 3 is generally between about 1-100 mg or 1-25 mg. Ligands containing fatty acyl chains are generally administered at doses from about 1-500 mg, 100-500 mg, or 100-300 mg.

Compounds (ligands) as described herein can be used for the preparation of a medicament for use in any of the methods of treatment described herein.

### Pharmaceutical Compositions

The compounds described herein, can be incorporated into pharmaceutical compositions. Such compositions typically include the compound and a pharmaceutically acceptable carrier. As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include, without limitation, parenteral (e.g., intravenous, intradermal, subcutaneous), oral, intranasal (e.g., inhalation), transdermal, transmucosal, intrathecal, intracerebral ventricular (e.g., using an Omaya reservoir-shunt with in-line filter that is surgically placed into the cisternal space), and rectal administration. Potentially useful parenteral delivery systems for a composition include, without limitation, slow-dissolving polymer particles, implantable infusion systems, and liposomes. Solutions or suspensions used for parenteral application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. Other appropriate solutions or suspensions can be used. pH can be adjusted with acids or bases, such as hydrochloric acid or sodium hydroxide. The parenteral preparation can be, for example, enclosed in ampoules, disposable syringes or multiple dose vials made of glass or plastic.

Treatment of a disorder related to an Aβ peptide, e.g., the undesirable production of an Aβ peptide or the production of fibrils comprising such peptides may also be effected by direct delivery of a compound described herein to the central nervous system, e.g., to the brain.

Pharmaceutical compositions suitable for injectable use include, without limitation, sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating on particles of the active substance (e.g., lecithin), by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it is preferable to include isotonic agents in the composition. Examples of such agents include sugars, polyalcohols (e.g., mannitol and sorbitol), and sodium chloride. Prolonged absorption of the injectable compositions can be effected by including in the composition an agent that delays absorption, for example, aluminum monostearate or gelatin. In general, methods for making such pharmaceutical compositions are known in the art.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the methods of preparation are generally vacuum drying and freeze-drying, which yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used for example, in the form of tablets, troches, or capsules, e.g., gelatin capsules. Pharmaceutically compatible binding agents, and/or adjuvant materials can be included as part of the composition. The tablets, pills, capsules, troches, and the like can contain other ingredients that are known in the art, e.g., the following ingredients, or ingredients of a similar nature: a binder such as microcrystalline cellulose, gum tragacanth or gelatin; an excipient such as starch or lactose; a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from pressured container or dispenser that contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are generally used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art and materials are commercially available, e.g., Alza Corporation and Nova Pharmaceuticals, Inc. Liposomal suspensions (including liposomes targeted to cells specifically affected by Aβ peptides such as neurons or glia with monoclonal antibodies or fragments thereof) can also be used as pharmaceutically acceptable carriers. These compositions can be prepared according to methods known to those skilled in the art, for example, as described in U.S. Pat. No. 4,522,811.

It is advantageous to formulate oral or parenteral compositions in dosage unit form for ease of administration and uniformity of dosage. Dosage unit form as used herein refers to physically discrete units suited as unitary dosages for the subject to be treated, each unit containing a predetermined quantity of active compound calculated to produce the desired therapeutic effect in association with the required pharmaceutical carrier.

Toxicity and therapeutic efficacy of compounds can be determined by known pharmaceutical procedures in cell cultures or experimental animals, e.g., for determining the LD50 (the dose lethal to 50% of the population) and the ED50 (the dose therapeutically effective in 50% of the population). Suitable animal models can be used such as those described for Aβ-associated conditions including animal models generated using genetic engineering (such as rodents engineered to express a human Aβ peptide) or naturally occurring conditions. Examples of animal models are described *supra* and also include Sturchler-Pierrat et al. (1999, Rev. Neurosci. 10:15-24), Seabrook et al. (1999, Neuropharmacol. 38:1-17), DeArmond et al. (1995, Brain Pathology 5:77-89), Telling (2000, Neuropathol. Appl. Neurobiol. 26:209-220), and Price et al. (1998, Science 282:1079-1083). The dose ratio between toxic and therapeutic effects is the therapeutic index and it can be expressed as the ratio LD50/ED50. Compounds that exhibit high therapeutic indices are preferred. While compounds that exhibit toxic side effects may be used, care should be taken to design a delivery system that targets such compounds to the site of affected tissue in order to minimize potential damage to unaffected cells and thereby reduce side effects.

Data obtained from the cell culture assays and animal studies can be used in formulating a range of dosage for use in humans. The dosage of a compound generally lies within a range of circulating concentrations that include the ED50 with little or no toxicity. The dosage may vary within this range depending upon the dosage form employed and the route of administration utilized. For any compound used in a method described herein, the therapeutically effective dose can be estimated initially from cell culture assays in which, e.g., the rate of fibril formation or the rate of cell death is observed. A dose can be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of a compound described herein (i.e., an effective dosage) ranges from about 0.001 to 100 mg/kg body weight, for example, about 0.01 to 25 mg/kg body weight, about 0.05 to 20 mg/kg body weight, about 0.1 to 10 mg/kg body weight, or about 20-100 mg/kg body weight. The compound can be administered over an extended period of time to the subject, e.g., over the subject's lifetime. In some cases the compound can be administered one time per week for between about 1 to 10 weeks, for example, between 2 to 8 weeks, between about 3 to 7 weeks, or for about 4, 5, or 6 weeks. The compound can also be administered chronically. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a compound can include a single treatment or, more generally, can include a series of treatments.

When one or more of these small molecules is to be administered to an animal (e.g., a mammal such as a human) to treat a disease associated with an Aβ peptide is involved (e.g., Alzheimer's disease), a physician, veterinarian, or researcher may, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

The pharmaceutical compositions can be included in a container, pack, or dispenser together with instructions for administration. For example, the instructions can include directions to use the composition to treat an individual having or at risk for an amyloidosis.

### Methods of Treatment

The present invention provides compounds of the invention for use in both prophylactic and therapeutic methods of treating a subject at risk for (or susceptible to) a disorder or having a disorder associated with an Aβ peptide, e.g. Alzheimer's disease *Dementia pugilistica,* severe head trauma, and certain pathologies and symptoms of Down syndrome. As used herein, the term "treatment" is defined as the application or administration of a therapeutic agent to a patient, or application or administration of a therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease or the predisposition toward disease. A therapeutic agent includes the compounds described herein.

Provided herein are compounds for preventing a disease or condition (i.e., decreasing the risk of contracting, or decreasing the rate at which symptoms appear that are associated with a disease or condition) associated with the presence of an Aβ peptide. Subjects at risk for a disease that is caused or exacerbated by such peptides can be identified by, for example, any or a combination of appropriate diagnostic or prognostic assays known in the art. Administration of a compound described herein as a prophylactic agent that can slow or prevent the pathology or other stigmata of an Aβ-associated disease can occur prior to the manifestation of symptoms characteristic of the disease, such that the disease is prevented or, alternatively, delayed in its progression.

The compounds described herein that are useful for treating or preventing an Aβ-related disease can be administered to a patient at therapeutically effective doses to prevent, treat, or ameliorate disorders involving an Aβ peptide. A therapeutically effective dose refers to that amount of the compound sufficient to result in amelioration of symptoms of the disorders or to slow or prevent the appearance of such symptoms. Toxicity and therapeutic efficacy of such compounds can be determined by pharmaceutical procedures as described above and that are known in the art.

### EXAMPLES

### Example 1: Design of Compounds and Molecular Dynamics Simulations of Aβ-Ligand Complexes

The amino acid sequence of human Aβ from residues 12 to 28 (Aβ12-28), including the discordant region between residues 16 and 23, was built into an α-helical conformation using molecular graphics (Insight II, Accelrys Inc., San Diego, CA). Based on the structure of the Aβ discordant helix, compounds with chemical structures complementary to the surface of the Aβ helix were designed using the Insight II system. In general, it was found that compounds useful for decreasing loss of α-helical conformations of Aβ are compounds that have one or more sites that can interact with (referring to Aβ peptides) at least two of the following; the side chain of Lys16, the side chain of His13, the side-chain of Glu23, the side chain of Phe19, the side chain of Phe20, and the side-chain of Asp22.

Fig. 5 shows the chemical structure of ligand 3 (a specific variant of Formula 1) and identifies functional groups that were designed to interact with specific portions of the Aβ peptide, provided that the corresponding portions of Aβ are in an α-helical conformation. Fig. 2 diagrams the Aβ-ligand 3 complex with the helical Aβ(12-28) peptide bound to ligand 3 via the interactions illustrated in Fig. 1. Importantly, Fig. 2 depicts the Aβ-ligand complex after it has undergone a 20 ps molecular dynamics (MD) simulation (at 37°C using Discover (Accelrys Inc.) and the Amber force field), starting with Aβ12-28 in α-helical conformation. The alpha-helical structure of the Aβ12-28 peptide is illustrated in Fig. 3. After the MD simulation period, the Aβ12-28 peptide shows α-helical structure, which is nearly identical to the α-helical structure before the start of the simulation. In sharp contrast, after a 20 ps MD simulation of Aβ12-28, starting from an α-helical conformation but in the absence of ligand, the Aβ12-28 peptide undergoes nearly complete unfolding. This is illustrated in Fig. 4.

### Example 2: Ligands

### Ligand 3

Ligand 3 (illustrated in Fig. 5), a specific example of Formula 1 (ligand 1; Fig. 1), was synthesized by solution synthesis using activated pentafluorophenyl esters. During the synthesis, known protecting groups, and standard coupling and deprotection protocols were used. All protected amino acids were purchased from Bachem (King of Prussia, PA). All other reagents were of commercial grade, and all solvents were of analytical grade, and were purchased from Fluka (St. Louis, MO), Merck (Whitehouse Station, NJ), or Aldrich Chemical Co (St. Louis, MO).

The synthesis of ligand 3 was started from α-N-benzyloxycarbonyldiaminobutyric acid (Z-Dab-OH). In the first step Fmoc-D-Trp-OH was coupled to Z-Dab-OH (at the γ-amino group), which resulted in a dipeptide. After removal of the Fmoc-group from the tryptophan derivative dipeptide, the next amino acid, Boc-Arg(Pbf)-OH, was coupled to the free α-amino group of the tryptophan derivative resulting in a tripeptide product. In the next step the Z-group was removed from the tripeptide, Z-Dab-(Boc-Arg(Pbf))-*D*-Trp)-OH, by catalytic hydrogenization using Pd/C. Fmoc-*D*-Glu(OtBu)-OH was subsequently coupled to the deprotected tripeptide at the α-amino group of Dab. The Fmoc-group in the resulting tetrapeptide was removed and the α-amino-group of the *D*-glutamate (D-Glu) residue was acetylated using acetic acid anhydride in pyridine. Final deprotection of the Boc, OtBu, and Pbf groups was performed with a deprotecting cocktail containing trifluoroacetic acid (TFA), thioanisol, phenol, and water. The crude peptide was purified by reversed-phase HPLC using a semi preparative column, detection at 220 nm, and a linear gradient of water/acetonitrile containing 0.1% TFA (0-80% acetonitrile over 2 hours at a flow rate of 3.5 ml/minute). The purity and structure of the final peptide ligand was determined by analytical HPLC and electrospray (ES) mass spectrometry.

Additional details of the synthesis are provided below. *Z-Dab(Fmoc-D-Trp)-OH*: 2.52 g (10.00 mmol) of Z-Dab-OH was dissolved in 50 ml of 50 mM Na₂B₄O₇ (pH 8.5). 5.93 g (10.00 mmol) of Fmoc-*D*-Trp-OPfp was dissolved in 50 ml of cold DMF (0°C) and added slowly to the solution of Z-Dab-OH. The mixture was stirred at 0°C for 1 hour and then for 10 hours at room temperature. The reaction was monitored by thin layer chromatography (TLC). After completion of the reaction, the solvent (H-₂O/DMF) was evaporated under reduced pressure. The oily product Z-Dab (Fmoc-D-Trp)-COOH was crystallized from ethyl acetate, collected, and dried under reduced pressure. The yield from this process was 5.94 g (90%).
*Z-Dab(D-Trp)-OH:* Z-Dab(Fmoc-D-Trp)-OH was treated with a solution of 20% piperidine in DMF (0.5 hours). The reaction mixture was concentrated and the oily product was crystallized from ether. The product was filtered off, washed with ether and dried under reduced pressure. The yield from this process was 98%.

*Z-Dab(Boc-Arg(Pbf)-D-Trp)-OH*: 0.93 g (2.18 mmol) of Z-Dab(D-Trp)-OH was dissolved in 30 ml of DMF and 1.5 g of Boc-Arg (Pbf)-OPfp (2.2 mmol), dissolved in cold DMF, was added slowly. The reaction mixture was stirred for 6 hours at room temperature. The reaction mixture was concentrated and the oily material was washed with cold ether. Precipitation occurred and the mixture was kept in the freezer for one hour. The precipitate was filtered out, washed with ether, and dried under reduced pressure. The yield from this process was 0.70 g.

*NH₂-Dab(Boc-Arg(Pbf)-D-Trp)-OH*: The Z-group was removed from Z-Dab(Boc-Arg(Pbf)-D-Trp)-OH by catalytic hydrogenation using H₂ and Pd/C in DMF, containing AcOH. The Pd/C catalyst was filtered off and the solution was evaporated under reduced pressure. The resulting oily product was worked up by the addition of ether. Precipitation occurred and the mixture was kept in the freezer, filtered off, washed with ether and dried under reduced pressure. 0.46 g (0.53 mmol) of the product was dissolved in a mixture of EtOAc/DMF and 53 ml (0.53 mmol) of N-methylmorpholine was added. The reaction was stirred for one hour, the liquid was evaporated and the peptide with free amino group was precipitated with ether.

*Fmoc-D-Glu(OtBu)-Dab(Boc-Arg(Pbf)-D-Trp)-OH*: 0.46 g (0.53 mmol) of NH₂-Dab(Boc-Arg(Pbf)-D-Trp)-OH was dissolved in DMF. 344.5 mg (0.58 mmol) of Fmoc-*D*-Glu(OtBu)-OPfp was dissolved in cold DMF and was added slowly at 0°C. The reaction mixture was stirred at 0°C for one hour, then 8 hours at room temperature. The solvent was evaporated and the product was dissolved in ether.

*Ac-D-Glu(OtBu)-Dab(Boc-Arg(Pbf)-D-Trp)-OH*: The Fmoc group was removed from 0.57 g of Fmoc-D-Glu(OtBu)-Dab(Boc-Arg(Pbf)-D-Trp)-OH by treatment with a solution of 20% piperidine in DMF for 0.5 hours. The peptide was then acetylated in pyridine containing 20% acetic anhydride for 15 minutes. The reaction mixture was concentrated and dissolved in ether. The yield of this process was 0.40 g.

*Ac-D-Glu-Dab(Arg-D-Trp)-OH:* Ac-D-Glu(OtBu)-Dab(Boc-Arg(Pbf)-D-Trp)-OH was treated with a cleavage cocktail containing 10 ml TFA, 1 ml H₂O, 0.75 g phenol, and 0.5 ml of thioanisole. The reaction was stirred for one hour at room temperature. Deprotection was monitored by TLC. After completion the reaction mixture was concentrated and ether was added. The mixture was stored in the freezer, then the formed solid was filtered off, washed with ether, and dried under reduced pressure. The yield of this process was 0.35 g of crude peptide. Purification of the crude peptide was accomplished using reverse phase HPLC on a Vydac semi-preparative column using a gradient of acetonitrile/water, containing 0.1% TFA (0-80% acetonitrile over two hours at a flow rate of 3.5 ml/min). The purity and molecular weight of the final peptide was determined by analytical HPLC and ESI-MS (electrospray mass spectroscopy analysis (Micromass LCT™, Waters, Millford, MA). MS (ESI, M+1): m/z found: 632.7; calculated: 632.3.

### Example 3: Effect of Ligands on Aβ Secondary Structure

Compounds described herein can be tested for their ability to affect the secondary structure of an Aβ peptide. As an example, addition of ligands to Aβ(1-40) or Aβ(12-28) in solution increased the helical content of Aβ peptides, as measured by circular dichroism (CD) spectroscopy. This is evidenced by a decrease in molar ellipticity at 208 and 222 nm, and an increase in molar ellipticity at 190-195 nm (an example is shown in Fig. 6). In these experiments, Aβ peptides (concentration about 20 µM to 100 µM) were mixed with ligands in ligand/Aβ molar ratios ranging between 1 and 10. The measurements were performed in phosphate buffer (as described below for thioflavin T measurements) or in phosphate buffer containing 30% trifluoroethanol. The samples were incubated at 37°C with or without shaking before measurements. CD measurements were performed at room temperature in a Jasco 810 instrument using a 1 mm cuvette (sample volume 200-300 µl). For each sample, three spectra were accumulated. Spectra were measured between 260 and 185 nm, and the spectra of buffer and ligands alone were subtracted from the spectra of the mixtures. Molar ellipticity values were calculated using the recorded spectra and the peptide concentrations, and expressed in kdeg x cm²/dmol. After addition of 10-30% (v/v) of trifluoroethanol to the Aβ peptide solutions, which increases the population of α-helical Aβ peptides, the increase in helical content by the ligands is more pronounced. These data indicate that that the tested ligands bind to helical Aβ, as predicted from their design.

In addition to verifying the usefulness of the tested ligands, this demonstrates the efficacy of using the methods described herein to design effective ligands.

### Example 4: Verification that Ligands Bind to Aβ Peptides

Experiments were performed to further demonstrate the binding of the ligands to Aβ. In these experiments, shifts in fluorescence emission were used to indicate binding. In these experiments, ligand 3 (about 10 µM) was dissolved in sodium phosphate buffer, pH 7, with or without trifluoroethanol present. Fluorescence was measured in a Hitachi F-4000 spectrophotometer, and emission was measured between 300 and 430 nm after excitation at 280 nm.

Ligand 3 exhibited fluorescence emission after excitation with light at 280 nm because of the presence of a tryptophan residue in those ligands (Figure 5). When Aβ(1-40) or Aβ(12-28) was added to a solution containing ligand 3, the fluorescence yield was increased (e.g., Fig 7) and the wavelength at maximum emission was shifted from about 348 nm to 344 nm.

The changes in fluorescence emission in a solution containing the ligands in the presence of Aβ demonstrate that the ligands and Aβ peptides containing the discordant helix form a complex in solution. As with the effects of the ligands on Aβ secondary structure, the fluorescence shifts are less pronounced in the absence of trifluoroethanol. This confirms that the ligands bind preferentially to Aβ peptides in α-helical conformation. These data also demonstrate that fluorescence emission methods can be used to test a ligand for the ability to interact with an Aβ peptide.

### Example 5: Effects of Ligands on Aβ Fibril Formation

Fibril formation of Aβ(1-40) or Aβ(12-28) with and without the presence of the ligands was assessed by the fibril-binding dye thioflavin T (ThT). Solutions containing Aβ peptides in concentrations from about 20 µM to 200 µM without ligand present, or at different peptide/ligand ratios were prepared in 10 mM sodium phosphate buffer, pH 7. The samples were incubated at 37°C under constant gentle shaking. At different time points aliquots were removed and ThT measurements performed using 10 µM ThT in 10 mM sodium phosphate buffer, pH 6, containing 150mMNaCl.

The samples were incubated for 10-15 minutes in the dark before ThT fluorescence measurements were made. Fluorescence was measured using a FarCyte™ (Tecan) instrument with excitation at 440 nm and emission at 480 nm. All samples were measured in duplicates.

In the presence of amyloid fibrils, ThT fluorescence is increased. In the absence of ligand, incubation of Aβ(1-40) resulted in an increased ThT fluorescence one to two hours after dissolution and within about two to three hours, maximum ThT fluorescence was reached. In the presence of ligand 3, the ThT fluorescence induced by Aβ(1-40) was practically abolished (Fig. 8). Ligand 3 was effective in reducing Aβ(1-40) fibril formation, as measured by ThT binding, already at a 1:1 ligand/peptide molar ratio (Fig. 9). Increasing the ligand/Aβ ratio to 5 resulted in further reduction of ThT fluorescence, while a ligand/Aβ ratio of 10 gives approximately the same result as a ligand/Aβ ratio of 5. In contrast to the reduction of Aβ fibril formation obtained by ligand 3, the dipeptide Arg-Gly, which was not expected to stabilize the Aβ discordant helix, had no effect on Aβ fibril formation (Fig. 10).

### Example 6: Effects of Ligand 3 In Vivo

### Ligand 3 increases longevity in a Drosophila melanogaster model of Alzheimer's disease

The *in vitro* results for ligand 3 (Ac-D-Glu-Dab(Arg-D-Trp)), demonstrate reduced Aβ aggregation in the presence of the ligand, which prompted investigation of the effects of ligand 3 in an *in vivo* model. A model of Alzheimer's disease was used in which *Drosophila melanogaster* express Aβ₁₋₄₂ in neurons (Crowther et al., 2005, Neuroscience 132:123-135). To evaluate the effect of ligand 3, longevity was observed in *Drosoplaila* expressing two copies of the Aβ₁₋₄₂ gene. These Aβ-expressing flies exhibit a markedly reduced survival compared to wild type flies (Crowther et al. 2005 *supra*).

In these experiments, Aβ-expressing flies were fed ligand 3 from the larval stage, which resulted in a significant increase in longevity compared to untreated flies under two different experimental conditions. For flies kept at 28°C and low humidity, 650 µM of ligand 3 in the food prolonged mean survival time (Fig. 11). Likewise, for flies kept at 29°C and high humidity, 134 µM of ligand 3 in the food increased the mean survival time (Fig. 12). The effects of ligand 3 are specific to Aβ-expressing flies as demonstrated by the absence of a statistically significant difference in effects on mean survival (longevity) in control flies not expressing Aβ after feeding with 650 µM of ligand 3 under the same conditions as described above (Fig. 13).

These data suggest a dose-response relationship for the survival effects induced by ligand 3 that is associated with ameliorating the deleterious effects of Aβ.

In general, these data demonstrate a positive effect of ligand 3 on survival in an organism expressing an abnormal amyloid polypeptide that is associated with Alzheimer's disease.

## Claims

1. A compound of formula 1 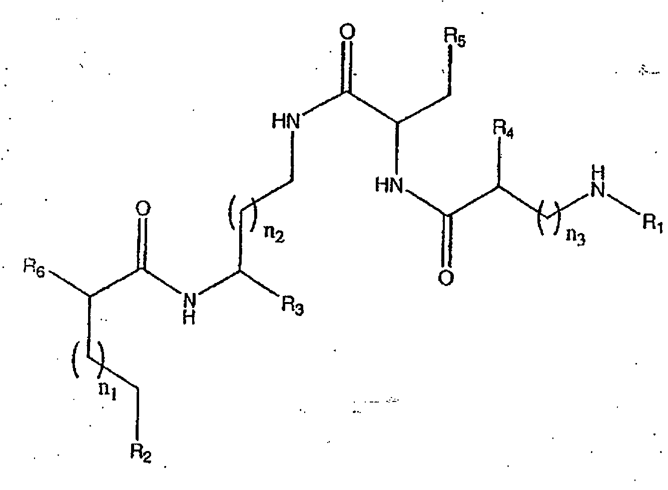 and pharmaceutically acceptable salts and hydrates thereof, wherein
n₁ is (CH₂)₁₋₄;
n₂ is (CH₂)₁₋₄;
n₃ is (CH₂)₁₋₄;
R₁ is an amine, diamine, -C=NH(NH₂), or carboxylamide; R₂ is carboxylic acid, phosphate, phosphonate, phosphinate, sulfate, or sulfonate;
R₃ is carboxylic acid, phosphate, phosphonate, phosphinate, sulfate, or sulfonate;
R₄ is an amine, diamine, carboxylic acid, or carboxylamide;
R₅ is an aromatic moiety; and
R₆ is not present or is a carboxylamide or any other group that does not interfere with the association of the compound with the Aβ peptide.

2. A compound of formula 1 having at least one of the following;
R₁ is -C=N(NH2).
R₂ is -C(O)OH.
R₃ is -C(O)OH.
R₄ is an amine.
R₅ is an aromatic ring associated with tryptophan.
R₆ is -NHC(O)CH₃.

3. A compound of formula 1 as claimed in claim 1 that can interact with at least two of
a. the side chain of Lysl6 of an Aβ peptide;
b. the side chain of Hisl3 of an Aβ peptide;
c. the side-chain of Glu23 of an Aβ peptide;
d. the side chain of Phel9 of an Aβ peptide;
e. the side chain of Phe20 or an Aβ peptide; and
f. the side-chain of Asp22 of an Aβ peptide;
and pharmaceutically acceptable salts thereof.

4. The compound of claim 3, wherein the compound can interact with at least three of a-f.

5. The compound of claim 3, wherein the compound can interact with at least four of a-f.

6. The compound of any one of claim 1, claim 2 or claim 3, wherein the compound can decrease the loss of α-helix in an Aβ peptide.

7. A compound comprising formula 1, wherein
n1 is (CH₂)₁₋₄, n2 is (CH₂)₁₋₄; n3 is (CH₂)₁₋₄;
R1 is an amine, diamine, or carboxylamide;
R2 is carboxylic acid, phosphate, phosphonate, phosphinate, sulfate, or sulfonate;
R3 is carboxylic acid, phosphate, phosphonate, phosphinate, sulfate, or sulfonate;
R4 is an amine, diamine, carboxylic acid, or carboxylamide;
R5 is an aromatic ring associated with tyrosine, phenylalanine, or tryptophan; and
R6 is not present or is a carboxylamide or any other group that does not interfere with the association of the compound with the Aβ peptide.

8. Ligand (3) having the formula

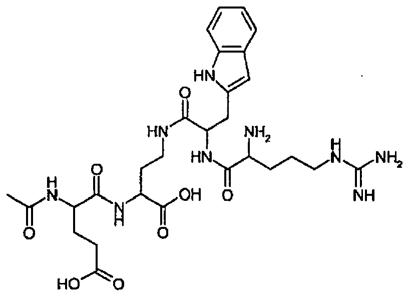

9. A composition comprising at least one compound of claim 1, claim 5 or claim 6, and a pharmaceutically acceptable excipient, diluent, or carrier.

10. A method of decreasing the loss of an Aβ peptide in an α-helical form, the method comprising contacting an Aβ peptide with a compound of claim 1, claim 5, or claim 6 with the proviso that the method is not a method for treatment of the human or animal body by surgery, or therapy and diagnostic methods practiced on the human or animal body.

11. A method of decreasing the amount of Aβ in a β form, the method comprising contacting an Aβ peptide with a compound of claim 1, claim 5, or claim 6 with the proviso that the method is not a method for treatment of the human or animal body by surgery, or therapy and diagnostic methods practiced on the human or animal body.

12. A method of decreasing the formation of Aβ aggregates, the method comprising contacting an Aβ peptide with a compound of claim 1, claim 5, or claim 6 with the proviso that the method is not a method for treatment of the human or animal body by surgery, or therapy and diagnostic methods practiced on the human or animal body.

13. A method of identifying a compound that is a candidate compound for treating an Aβ peptide-related disorder, the method comprising;
a) providing a compound of claim 1, claim 2, claim 3, claim 5, or claim 6;
b) contacting an Aβ peptide with the compound, thereby providing a sample;
c) determining the amount of Aβ peptide in α-helical form or β form in the sample;
d) comparing the amount of Aβ peptide in α-helical form or β form compared to a reference, wherein a compound that increases the amount of Aβ peptide in α-helical form or decreases the amount of Aβ peptide in β form in the sample compared to the reference is a candidate compound.

14. The method of claim 13, wherein the Aβ peptide is provided *in vitro.*

15. The method of claim 13, wherein the amount of Aβ peptide in β form is determined using Thioflavin T.

16. A therapeutically effective amount of a compound of claim 1, claim 2, claim 3, claim 5, or claim 6 for use in treating an Aβ peptide-related disorder.

17. The compound of claim 16, wherein the Aβ peptide-related disorder is Alzheimer's disease.

18. The compound of claim 1, claim 5 or claim 6 for use in decreasing the loss of an Aβ peptide in an alpha-helical form.

19. The compound of claim 1, claim 5 or claim 6 for use in decreasing the amount of Aβ peptide in a beta form.

20. The compound of claim 1, claim 5 or claim 6 for use in decreasing the formation of Aβ aggregates.

## Patentansprüche

1. Verbindung der Formel 1 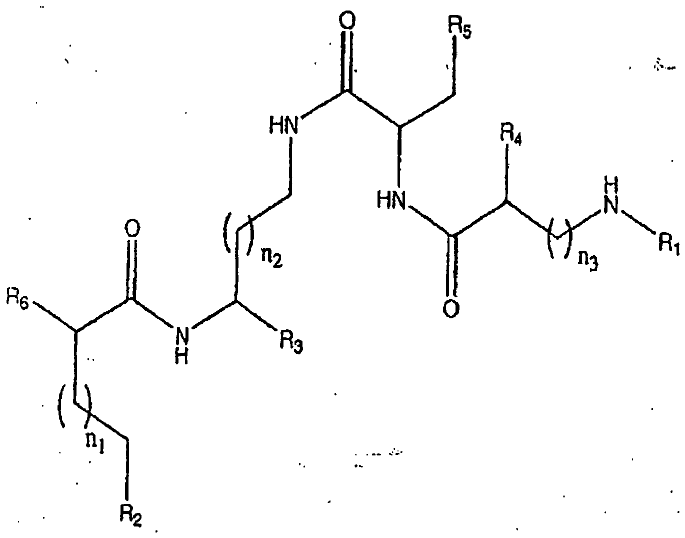 und pharmazeutisch annehmbare Salze und Hydrate davon, worin
n₁ für (CH₂)₁₋₄ steht;
n₂ für (CH₂)₁₋₄ steht;
n₃ für (CH₂)₁₋₄ steht;
R₁ für ein Amin, Diamin, -C=NH(NH₂) oder Carboxylamid steht;
R₂ für Carbonsäure, Phosphat, Phosphonat, Phosphinat, Sulfat oder Sulfonat steht;
R₃ für Carbonsäure, Phosphat, Phosphonat, Phosphinat, Sulfat oder Sulfonat steht;
R₄ ein Amin, Diamin, Carbonsäure oder Carboxylamid darstellt;
R₅ eine aromatische Komponente darstellt; und
R₆ nicht vorhanden ist oder für ein Carboxylamid oder eine andere Gruppe steht, die die Assoziation der Verbindung mit dem Aß-Peptid nicht stört.

2. Verbindung der Formel 1 mit mindestens einem der folgenden:
R₁ steht für -C=N(NH2);
R₂ steht für -C(O)OH;
R₃ steht für -C(O)OH;
R₄ steht für ein Amin;
R₅ steht für einen aromatischen Ring, assoziiert mit Tryptophan;
R₆ steht für -NHC(O)CH₃.

3. Verbindung der Formel 1 wie in Anspruch 1 beansprucht, die mit mindestens zweien von
a. der Seitenkette von Lys16 eines Aß-Peptids;
b. der Seitenkette von His13 eines Aß-Peptids;
c. der Seitenkette von Glu23 eines Aß-Peptids;
d. der Seitenkette von Phe19 eines Aß-Peptids;
e. der Seitenkette von Phe20 eines Aß-Peptids; und
f. der Seitenkette von Asp22 eines Aß-Peptids interagieren kann, und pharmazeutisch annehmbare Salze davon.

4. Verbindung nach Anspruch 3, wobei die Verbindung mit mindestens dreien von a-f interagieren kann.

5. Verbindung nach Anspruch 3, wobei die Verbindung mit mindestens vieren von a-f interagieren kann.

6. Verbindung nach einem von Anspruch 1, Anspruch 2 oder Anspruch 3, wobei die Verbindung den Verlust von α-Helix in einem Aß-Peptid verringern kann.

7. Verbindung umfassend Formel 1, worin
n1 für (CH₂)₁₋₄ steht, n2 für (CH₂)₁₋₄ steht; n3 für (CH₂)₁₋₄ steht;
R1 für ein Amin, Diamin oder Carboxylamid steht;
R2 für Carbonsäure, Phosphat, Phosphonat, Phosphinat, Sulfat oder Sulfonat steht;
R3 für Carbonsäure, Phosphat, Phosphonat, Phosphinat, Sulfat oder Sulfonat steht;
R4 für ein Amin, Diamin, Carbonsäure oder Carboxylamid steht;
R5 für einen aromatischen Ring, assoziiert mit Tyrosin, Phenylalanin oder Tryptophan steht; und
R6 nicht vorhanden ist oder für ein Carboxylamid oder eine andere Gruppe steht, die die Assoziation der Verbindung mit dem Aß-Peptid nicht stört.

8. Ligand (3) mit der Formel

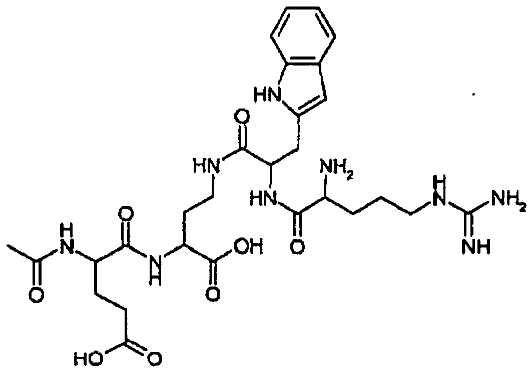

9. Zusammensetzung, welche mindestens eine Verbindung nach Anspruch 1, Anspruch 5 oder Anspruch 6 und einen pharmazeutisch annehmbaren Arzneimittelträger, Verdünnungsmittel oder Träger umfasst.

10. Verfahren zum Verringern des Verlustes eines Aß-Peptids in einer α-helikalen Form, wobei das Verfahren Kontaktieren des Aβ-Peptids mit einer Verbindung nach Anspruch 1, Anspruch 5 oder Anspruch 6 umfasst, mit der Maßgabe, dass das Verfahren kein Verfahren zur Behandlung des menschlichen oder tierischen Körpers durch Chirurgie ist, oder Therapie und diagnostische Verfahren, welche am menschlichen oder tierischen Körper praktiziert werden.

11. Verfahren zum Verringern der Menge an Aβ in einer β-Form, wobei das Verfahren Kontaktieren eines Aß-Peptids mit einer Verbindung nach Anspruch 1, Anspruch 5 oder Anspruch 6 umfasst, mit der Maßgabe, dass das Verfahren kein Verfahren zur Behandlung des menschlichen oder tierischen Körpers durch Chirurgie ist, oder Therapie und diagnostische Verfahren, welche am menschlichen oder tierischen Körper praktiziert werden.

12. Verfahren zum Verringern der Bildung von Aß-Aggregaten, wobei das Verfahren Kontaktieren eines Aß-Peptids mit einer Verbindung nach Anspruch 1, Anspruch 5 oder Anspruch 6 umfasst, mit der Maßgabe, dass das Verfahren kein Verfahren zur Behandlung des menschlichen oder tierischen Körpers durch Chirurgie ist, oder Therapie und diagnostische Verfahren, welche am menschlichen oder tierischen Körper praktiziert werden.

13. Verfahren zum Identifizieren einer Verbindung, die ein Verbindungskandidat zum Behandeln einer mit Aß-Peptid in Beziehung stehenden Störung ist, wobei das Verfahren umfasst:
a) Vorsehen einer Verbindung nach Anspruch 1, Anspruch 2, Anspruch 3, Anspruch 5 oder Anspruch 6;
b) Kontaktieren eines Aß-Peptids mit der Verbindung, dadurch Vorsehen einer Probe;
c) Bestimmen der Menge an Aß-Peptid in α-helikaler Form oder β-Form in der Probe;
d) Vergleichen der Menge an Aß-Peptid in α-helikaler Form oder β-Form verglichen mit einer Referenz, wobei eine Verbindung, die in der Probe verglichen mit der Referenz die Menge an Aß-Peptid in α-helikaler Form erhöht oder die Menge an Aß-Peptid in β-Form verringert, ein Verbindungskandidat ist.

14. Verfahren nach Anspruch 13, wobei das Aß-Peptid *in vitro* vorgesehen ist.

15. Verfahren nach Anspruch 13, wobei die Menge an Aß-Peptid in β-Form unter Verwendung von Thioflavin T bestimmt wird.

16. Therapeutisch wirksame Menge einer Verbindung nach Anspruch 1, Anspruch 2, Anspruch 3, Anspruch 5 oder Anspruch 6 zur Verwendung beim Behandeln einer mit Aß-Peptid in Beziehung stehenden Störung.

17. Verbindung nach Anspruch 16, wobei die mit Aß-Peptid in Beziehung stehende Störung Alzheimer-Krankheit ist.

18. Verbindung nach Anspruch 1, Anspruch 5 oder Anspruch 6 zur Verwendung beim Verringern des Verlustes eines Aß-Peptids in einer alpha-helikalen Form.

19. Verbindung nach Anspruch 1, Anspruch 5 oder Anspruch 6 zur Verwendung beim Verringern der Menge an Aß-Peptid in einer beta-Form.

20. Verbindung nach Anspruch 1, Anspruch 5 oder Anspruch 6 zur Verwendung beim Verringern der Bildung von Aß-Aggregaten.

## Revendications

1. Composé de formule 1 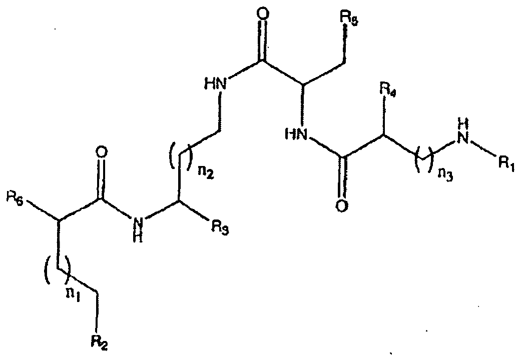 et sels et hydrates pharmaceutiquement acceptables de celui-ci, dans lequel
n₁ est (CH₂)₁₋₄ ;
n₂ est (CH₂)₁₋₄ ;
n₃ est (CH₂)₁₋₄ ;
R₁ est un groupe amine, diamine, -C=NH(NH₂) ou carboxylamide ;
R₂ est un groupe acide carboxylique, phosphate, phosphonate, phosphinate, sulfate ou sulfonate ;
R₃ est un groupe acide carboxylique, phosphate, phosphonate, phosphinate, sulfate ou sulfonate ;
R₄ est un groupe amine, diamine, acide carboxylique ou carboxylamide ;
R₅ est un groupe caractéristique aromatique ;
et
R₆ n'est pas présent ou est un groupe carboxylamide ou n'importe quel autre groupe qui n'interfère pas avec l'association du composé avec le peptide Aβ.

2. Composé de formule 1 ayant au moins un parmi les éléments suivants :
R₁ est un groupe -C=N(NH₂),
R₂ est un groupe -C(O)OH,
R₃ est un groupe -C(O)OH,
R₄ est un groupe amine,
R₅ est un cycle aromatique associé au tryptophane,
R₆ est un groupe -NHC(O)CH₃.

3. Composé de formule 1 selon la revendication 1 qui peut interagir avec au moins deux parmi
a. la chaîne latérale de Lys16 d'un peptide Aβ ;
b. la chaîne latérale d'His13 d'un peptide Aβ ;
c. la chaîne latérale de Glu23 d'un peptide Aβ ;
d. la chaîne latérale de Phe19 d'un peptide Aβ ;
e. la chaîne latérale de Phe20 d'un peptide Aβ ; et
f. la chaîne latérale d'Asp22 d'un peptide Aβ ;
et sels pharmaceutiquement acceptables de celui-ci.

4. Composé selon la revendication 3, le composé pouvant interagir avec au moins trois de a-f.

5. Composé selon la revendication 3, le composé pouvant interagir avec au moins quatre de a-f.

6. Composé selon l'une quelconque de la revendication 1, de la revendication 2 ou de la revendication 3, le composé pouvant réduire la perte d'hélice α dans un peptide Aβ

7. Composé de formule 1, dans lequel
n1 est (CH₂)₁₋₄ ; n2 est (CH₂)₁₋₄ n3 est (CH₂)₁₋₄ ;
R1 est un groupe amine, diamine ou carboxylamide ;
R2 est un groupe acide carboxylique, phosphate, phosphonate, phosphinate, sulfate ou sulfonate ;
R3 est un groupe acide carboxylique, phosphate, phosphonate, phosphinate, sulfate ou sulfonate ;
R4 est un groupe amine, diamine, acide carboxylique ou carboxylamide ;
R5 est un cycle aromatique associé à un groupe tyrosine, phénylalanine ou tryptophane ; et
R6 n'est pas présent ou est un groupe carboxylamide ou n'importe quel autre groupe qui n'interfère pas avec l'association du composé avec le peptide Aβ.

8. Ligand (3) de formule

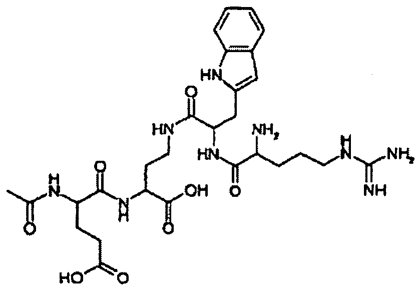

9. Composition comprenant au moins un composé selon la revendication 1, la revendication 5 ou la revendication 6, et un excipient, diluant ou support pharmaceutiquement acceptable.

10. Procédé de réduction de la perte d'un peptide Aβ dans une forme α-hélicoïdale, le procédé consistant à mettre en contact un peptide Aβ avec un composé selon la revendication 1, la revendication 5 ou la revendication 6 à condition que le procédé ne soit pas un procédé de traitement d'un corps humain ou animal par chirurgie, ou des procédés de thérapie et de diagnostic pratiqués sur le corps humain ou animal.

11. Procédé de réduction de la quantité de Aβ sous une forme β, le procédé consistant à mettre en contact un peptide Aβ avec un composé selon la revendication 1, la revendication 5 ou la revendication 6 à condition que le procédé ne soit pas un procédé de traitement du corps humain ou animal par chirurgie, ou des procédés de thérapie et de diagnostic pratiqués sur le corps humain ou animal.

12. Procédé de réduction de la formation d'agrégats Aβ, le procédé consistant à mettre en contact un peptide Aβ avec un composé selon la revendication 1, la revendication 5 ou la revendication 6 à condition que le procédé ne soit pas un procédé de traitement du corps humain ou animal par chirurgie, ou des procédés de thérapie et diagnostic pratiqués sur le corps humain ou animal.

13. Procédé d'identification d'un composé qui est un composé candidat pour le traitement d'une maladie liée au peptide Aβ, le procédé consistant à :
a) fournir un composé selon la revendication 1, la revendication 2, la revendication 3, la revendication 5 ou la revendication 6 ;
b) mettre en contact un peptide Aβ avec le composé, fournissant ainsi un échantillon ;
c) déterminer la quantité de peptide Aβ sous forme α-hélicoïdale ou forme β dans l'échantillon ;
d) comparer la quantité de peptide Aβ sous forme α-hélicoïdale ou forme β à une référence, où un composé qui augmente la quantité de peptide Aβ sous forme α-hélicoïdale ou réduit la quantité de peptide Aβ sous forme β dans l'échantillon comparé à la référence est un composé candidat.

14. Procédé selon la revendication 13, dans lequel le peptide Aβ est fourni in vitro.

15. Procédé selon la revendication 13, dans lequel la quantité de peptide Aβ sous forme β est déterminée en utilisant de la thioflavine T.

16. Quantité thérapeutiquement efficace d'un composé selon la revendication 1, la revendication 2, la revendication 3, la revendication 5 ou la revendication 6 destinée à être utilisée dans le traitement d'une maladie liée au peptide Aβ.

17. Composé selon la revendication 16, dans lequel la maladie liée au peptide Aβ est la maladie d'Alzheimer.

18. Composé selon la revendication 1, la revendication 5 ou la revendication 6 destiné à être utilisé pour réduire la perte d'un peptide Aβ sous une forme alpha-hélicoïdale.

19. Composé selon la revendication 1, la revendication 5 ou la revendication 6 destiné à être utilisé pour réduire la quantité de peptide Aβ sous une forme bêta.

20. Composé selon la revendication 1, la revendication 5 ou la revendication 6 destiné à être utilisé pour réduire la formation d'agrégats Aβ.
